# EUROPEAN PATENT APPLICATION

(11) **EP 1 469 076 A2**
(43) Date of publication of application: **20.10.2004**
(21) Application number: 04076580.2
(22) Date of filing: 10.05.1995
(51) Int. Cl.: C12N 15/40, C07K 14/08, C12N 7/01, A61K 39/12, G01N 33/569

(54) **Recombinant PRRS proteins, diagnostic kits and vaccines containing said recombinant proteins**

(30) Priority: 13.05.1994 ES 9401027; 27.04.1995 ES 9500815
(62) Divisional of application: 95917990.4
(71) Applicant: Wyeth Farma, S.A., 28700 San Sebastian de los Reyes, Madrid (ES)
(72) Inventor: Plana Duran, Juan, 17813 Vall de Bianya (ES); Casal Alvarez, José Ignacio, 28037 Madrid (ES); Climent Sanchez, Isabel, 17813 Vall de Bianya (ES)
(74) Representative: Wileman, David Francis Dr.

(57) **Abstract**

The present invention discloses the production of recombinant proteins of the virus causing the porcine respiratory and reproductive syndrome (PRRS), corresponding to the OFRₛ 2-7 of the PRRSV isolated in Spain, (PRRS-Olot), in a system of expression of recombinant baculoviruses multiplided in a cell culture of a *permissive host*. Said recombinant proteins are appropriate to formulate vaccines capable of efficiently protecting pigs'against PRRS as well as to prepare diagnostic kits appropriate to detect both the presence of antibodies which recognize PRRSV and the presence of PRRSV in a porcine biological sample. This invention applies to veterinary.

## Description

### SCOPE OF THE INVENTION

This invention relates to viral recombinant proteins of the causative agent of porcine reproductive and respiratory syndrome (PRRS) produced in an expression system of recombinant baculoviruses multiplied in permissive host cell culture. The invention also relates to diagnostic kits and vaccines which comprise, at least, one of the said recombinant proteins.

### HISTORY OF THE INVENTION

In Spain, the first cases of respiratory alterations in piglets were detected in a 300-piglet batch imported from Germany, in mid-January 1991 (Plana et al., Med. Vet., Vol. 8, No. 11, 1991). Shortly afterwards, in two breeding herds on two farms situated near the herd where the initial problem had appeared, a disease was detected characterized by an abnormally high number of abortions during the last phase of gestation, as well as 70% mortality in piglets.

The cause of these epizootic outbreaks was not known, but their symptomatology was similar to the clinical signs that had been described for a swine disease first detected in Europe in Germany (1991), and to the disease denominated Mystery Swine Disease detected in the United States and Canada in 1987 (Hill, Proceedings of the Mystery Swine Disease Committee Meeting, October 6, 1990, Denver, USA). This disease affects pregnant sows, provoking in them anorexia, abortions, stillbirths, mummified fetuses, weak piglets that die in a few hours of life, and post-farrowing respiratory problems, among others. At present, the disease is known as "Porcine Reproductive and Respiratory Syndrome" (PRRS), although it was previously referred to as "Blue-eared Pig Disease", "Mysterious Reproductive Syndrome" (MRS), "Swine Infertility and Respiratory Syndrome" (SIARS) and "Porcine Epidemic Abortion and Respiratory Syndrome" (PEARS).

At present, it is known that the causative agent of this disease is a virus donominated as PRRS virus (PRRSV). This virus was isolated for the first time in the Netherlands by a group of researchers of the CDI/Lelystad, who denominated it as Lelystad virus (LV) (Wesvoort, G et al., Vet. Quarterly, Vol 3, 121-130, 1991). Some months later, another isolate was obtained in Spain by Laboratorios Sobrino/Cyanamid (Plana et al., Vet. Microbiol., 33:203.211, 1992), which will be identified in this description as PRRS-Olot. From that time, new isolates of this virus have been described (EP Requests No. 0 529 584 P2, PCT Requests Nos. WO 93/06211 and WO 93/07898).

The structural characteristics of the PRRS virus have been described in two recent publications:
a) Meulenberg, J.J.M., et al., "Lelystad virus, the causative agent of porcine epidemic abortion and respiratory syndrome (PEARS), is related to LDV and EAV". Virology, 192: 62-72, (1993); and
b) Cozelmann, K-K., et al., "Molecular characterizarion of porcine reproductive and respiratory syndrome virus, a member of the Arterivirus group". Virology, 193: 329-339, (1993).

The PRRS virus has a size of 50-60 nm, with an envelope of of approximately 30-35 nm contained in the nucelocapsid, and a single RNA molecule as genomic material. Based on these morphological data, PRRSV was initially classified as a Togavirus, although based on its genomic structure and transcription and translation mechanisms it was closer to the Coronaviridae family. Recently, and based on differences and/or similarities in comparison with the previous groups, its classification was proposed within a new family denominated Arteriviridae (Cavanagh D., et al., Arch. Virology, 1994). Together with PRRSV, in this group are included the equine arteritis viruses (EAV), lactic dehydrogenase virus (LDV) and simian hemorrhagic fever virus (SHFV).

Recently, the entire Lelystad virus (LV) genome (Meulenberg et al., quoted above), a genomic segment of the Tübingen (Germany) PRRS virus isolate (TV) (Cozelmann et al., quoted above), and a segment of the PRRS-Olot virus (Spanish Patent claim no. ES P9301973) were cloned and sequenced. Based on all the results obtained it can be stated that the PRRSV genome is made up of a single strand RNA molecule which contains at 3' end a poly-A tail. The length of the genome is of approximately 15000 base pairs (bp), and in its structure it contains seven open reading frames (ORFs) coding for the viral proteins. The ORFs have been denominated as ORF1 to ORF7 and they show small overlapping segments between them. It has been propounded that synthesis of the viral proteins is produced from a group of different length subgenomic transcripts (mRNA), but of similar 3' polyadenilated end, and 5' leader sequence originating from the non-coding 5' end sequence. This form of viral protein expression has been denominated as nested mRNAs and has been previously described for coraniviruses (Spaan, W.J.M., Cavanagh, D., and Horzineck, M.C., J. Gen. Virol., 69:2939-2952, 1988). Based on the Lelystad (LV) and Tübingen (TV) PRRSV viral isolate nucleotide sequence, and by homology with what has been observed with other arteriviruses, it has been propounded that in the viral genome, ORF1 (a and b) code for viral polymerase and replicase. ORFs 2 to 6 would code for the viral envelope proteins, and ORF7 would code for the neuclocapsid protein. Viral replicase and polymerase are large-sized proteins, 260 and 163 kDa respectively, and both of them contain three possible glycosilation sites. Envelope proteins (ORFs 2 to 6) located at 3' end are small, between 30 and 19 kDa. All of them contain more than two possible glycosilation sites, especially ORF3 which contains 7 sites. All of these proteins contain hydrophobic sequences at the amino (N-) and carboxy (C-) terminal ends that might work as leader sequence and membrane anchor. Generally, they are hydrophobic proteins, in accordance with their location associated to a membrane. ORF6 should be pointed out, with 3 hydrophobic segments located within the 90 amino acid residues at the N-terminal end. On the other hand, the protein coded by ORF7, possibly corresponding to the viral nucleocapsid, is extremely basic with arginine, lysine and histidine residues at the N-terminal end. The amino acid sequences of LV and TV viral polymerase, structural proteins and nucleocapsid show an identity of between 29% and 67% in comparison with LDV virus, and between 20% and 36% in comparison with EAV virus. This suggests that the evolution of the PRRS virus is closer to LDV than to EAV.

The disease caused by PRRSV is responsible for severe losses to the pig industry. For this reason, vaccines capable of preventing the infection caused by PRRSV have been developed.

In general, the vaccines against known PRRSV, described in patent claims WO 92/21375, WO 93/06211, WO 93/07898 and ES P9301973 are vaccines obtained from viruses grown on macrophages and subsequently inactivated. Patent application ES P9301973 provides a vaccine capable of avoiding porcine reproductive and respiratory syndrome (PRRS) . The vaccine has been demonstrated to be efficacious in avoiding reproductive alterations in sows, such as the farrowing of stillborn, mummified or living but weak piglets, repetition of estrus and similar problems produced by the virus causative of PRRS. Likewise, it has been verified that the vaccine induces cellular immunity in the vaccinated animals. The said vaccine contains a suitable quantity of PRRS viral antigen, Spanish strain (PRRS-Olot), inactivated, together with an adjuvant and preservative.

The present invention provides a second generation vaccine in which recombinant DNA technology has been employed with the objective of obtaining new vaccines capable of efficaciously protecting against the infection caused by PRRSV. The vaccines of this invention contain, at least, one recombinant PRRSV protein. On the other hand, the present invention provides new PRRSV diagnostic systems or kits that involve the use of enzymatic immunoassay techniques (ELISA) that use recombinant PRRSV proteins. These recombinant vaccines do not require manipulation of the complete virus, but rather of only part of it, eliminating the risk of an accident that would free virus, representing a considerable advantage over the present inactivated PRRSV vaccines. These new recombinant vaccines do not require manipulation of the complete virus, but rather of only part of it, eliminating the risk of an accident that would free virus, which represents a considerable advantage over the present inactivated PRRSV vaccines.

The production of recombinant proteins by means of Genetic Engineering is a fact that has been described previously. Numerous expression and production systems of recombinant proteins are known. One of the most effective systems for large-scale production of recombinant proteins is based on the replication of recombinant baculoviruses derived from the *Autographa californica* nuclear polyhedrosis virus (AcNPV), in insect cells in culture. The description of the baculovirus expression technique is described in the following articles:
a) LucKow, V.A. & Summers, M.D., "Trends in the development of baculovirus expression vectors". Bio/Technology, 6:47-55, (1988) ; and
b) Bishop, D.H.L., "Baculovirus expression vectors". Seminars in VIROLOGY, 3:253-264 (1992).

This invention provides recombinant PRRSV proteins, in particular of the PRRS-Olot isolate, produced in an expression system of baculoviruses multiplied on permissive host cell culture. The recombinant baculoviruses capable of producing such recombinant proteins, as well as the transfer vectors used, constitute additional objectives of the invention. The procedures for the obtainment of such recombinant baculoviruses and proteins is also an objective of this invention.

The invention provides also new vaccines for the vaccination of pigs for their protection against the infection caused by PRRSV, comprising, at least, one recombinant protein of those provided by this invention and an adequate carrier or adjuvant.

The invention provides also a diagnostic kit to detect the presence of antibodies that specifically recognize PRRSV in a biological sample from pigs (e.g.: blood, serum, sputum, saliva or milk). The kit comprises at least one recombinant protein of those provided by this invention and adequate detection methods.

The invention provides also a diagnostic kit for the detection of the presence of antigen (PPRSV) in a biological sample from pigs (e.g.: blood, serum, sputum, saliva, milk or tissue). The kit comprises at least one antibody which specifically recognizes PRRSV obtained by immunizing animals with, at least, one recombinant protein of those provided by this invention and adequate detection means.

### BRIEF DESCRIPTION OF THE FIGURES

Figure 1 shows the consecutive sequence of the 3383 bp cloned from the PRRS-Olot isolate.
Figure 2 shows the amino acid sequence corresponding to the proteins coded by ORF2 (Figure 2A), ORF3 (Figure 2B), ORF4 (Figure 2C), ORF5 (Figure 2D), ORF6 (Figure 2E) and ORF7 (Figure 2F).
Figure 3 shows the different extension of clones pPRRS-8, pPRRS-108, pPRRS-121, pPRRS-132, pPPRS-146, pPPRS-147, pPPRS-148, pPRRS-153 and pPRRS-3, in comparison with LV, as well as the ORFs contained in each one of them. In this figure, reference is made to the PRRSV genome (a), size in Kb (b) and number of the clone (c).
Figure 4 shows pPRRS-3 clone containing the gene of the protein coded by ORF2.
Figure 5 shows pPRRS-121 clone containing the gene of the protein coded by ORF3.
Figure 6 shows pPRRS-146 clone containing the gene of the protein coded by ORF4.
Figure 7 shows pPRRS-132 clone containing the gene of the protein coded by ORF5.
Figure 8 shows pPRRS-8 clone containing the genes of the proteins coded by ORF6 and ORF7.
Figure 9 shows the results from antigen titration by ELISA (absorbance monitored at 405 nm). Figure 9 shows the results of antigen titration by ELISA. In the figure reference is made to antigen titration (a), absorbance values read at 405 nm (b), and antigen dilutions [in units of 1/ ] (c).
Figure 10 shows the results from the titration, by ELISA, of a PRRS field serum obtained in an infected animal. The figure makes reference to the titration of the serum (a), absorbance values read at 405 nm (b), and serum dilutions [in units of 1/ ] (c).
Figure 11 shows the results obtained from a sampling experiment with several dozen field sera. The figure makes reference to the titration of the sera (a), absorbance values read at 405 nm (b), and the sera (c)

### DESCRIPTION OF THE INVENTION

Our Laborotry has made a search for the PRRS causative agent in recent years. The main consequence of this has been the isolation of the virus denominated PRRS-CY-JPD-P5-6-91. It was deposited at the ECACC (with accession number V93070108) and a vaccine was developed against PRRSV containing the inactivated virus (Patent Application ES P9301973).

Since then, our research efforts have addressed the isolation and cloning of the PRRSV (PRRS-CY-JPD-P5-6-91) genome, denominated as PRRS-Olot in this description, in order to enable the development of new recombinant vaccines effective against the infection caused by PRRSV. To that end, a genome segment of the said PRRS-Olot genome has been cloned. The cloned fragment corresponds to the 3' viral genome, and represents a consecutive sequence of 3338 bp. This segment contains the six open reading frames corresponding to ORFs 2 to 7 described for LV and TV. They code for the structural proteins of the virus (nucleocapsid and envelope) possibly involved in viral antigenicity and immunogenicity. The proteins coded by PRRS-Olot ORFs 2 to 7 are similar to the corresponding LV and TV proteins. Their characteristics are summarized in Table 1, where are indicated, in relation with each ORF, the relative positions of the nucleotides, the number of base pairs (bp), the number of amino acids (Aac), the molecular weight of each protein (in KDa) and the glycosilation sites.

**Table 1**

| Characteristics of the PRRS-Olot virus ORFs | | | | | |
|---|---|---|---|---|---|
| ORF | Nucleotides (site) | bp | Aac (No.) | Protein (KDa) | Glycosilation |
| 2 | 65-811 | 747 | 249 | 28.4 | 2 |
| 3 | 673-1467 | 795 | 265 | 30.8 | 7 |
| 4 | 1215-1763 | 549 | 183 | 20.0 | 5 |
| 5 | 1763-2362 | 600 | 200 | 22.4 | 2 |
| 6 | 2353-2871 | 519 | 173 | 19.0 | 2 |
| 7 | 2864-3247 | 384 | 128 | 13.8 | 1 |

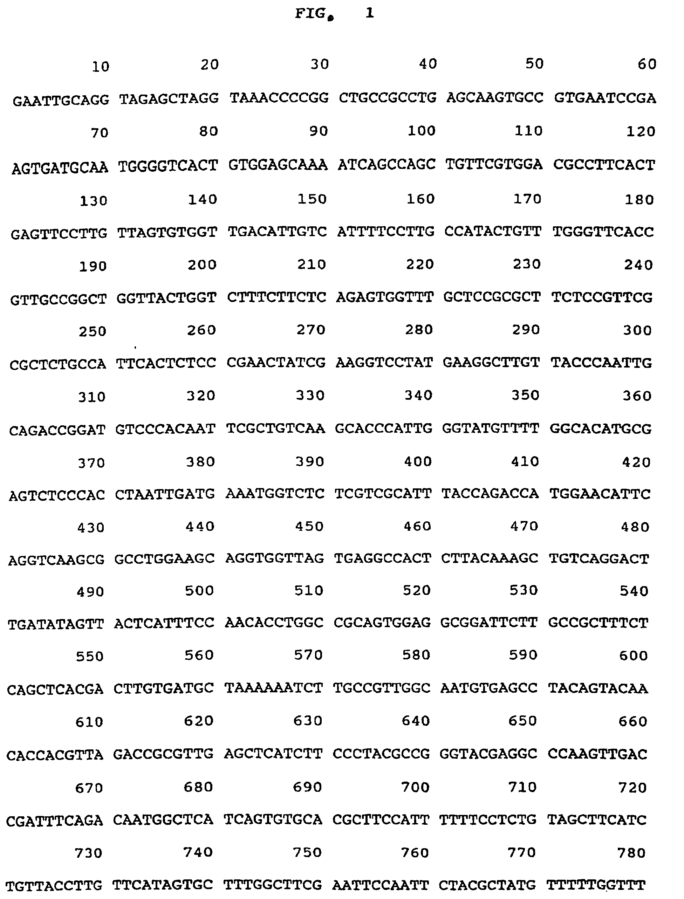

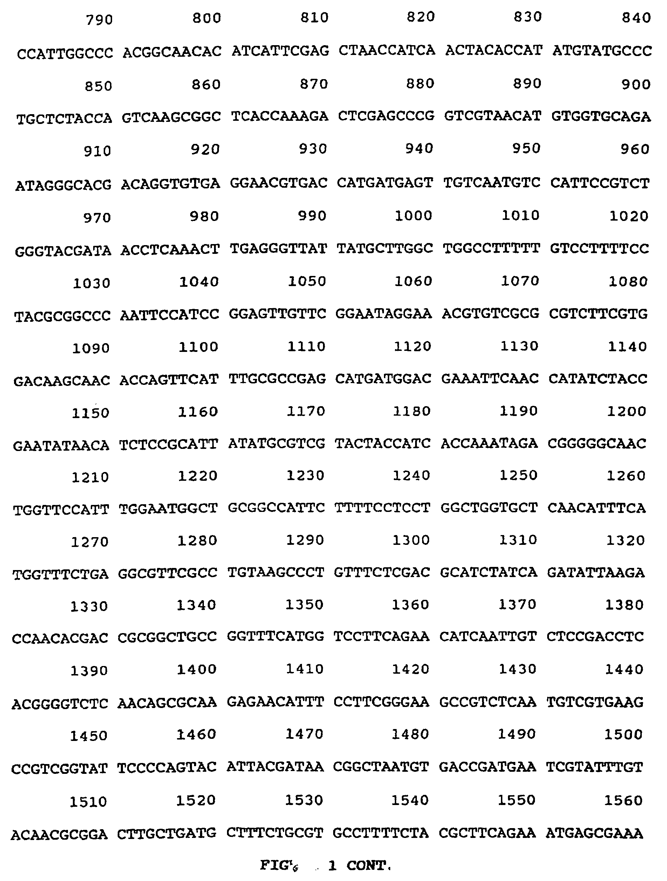

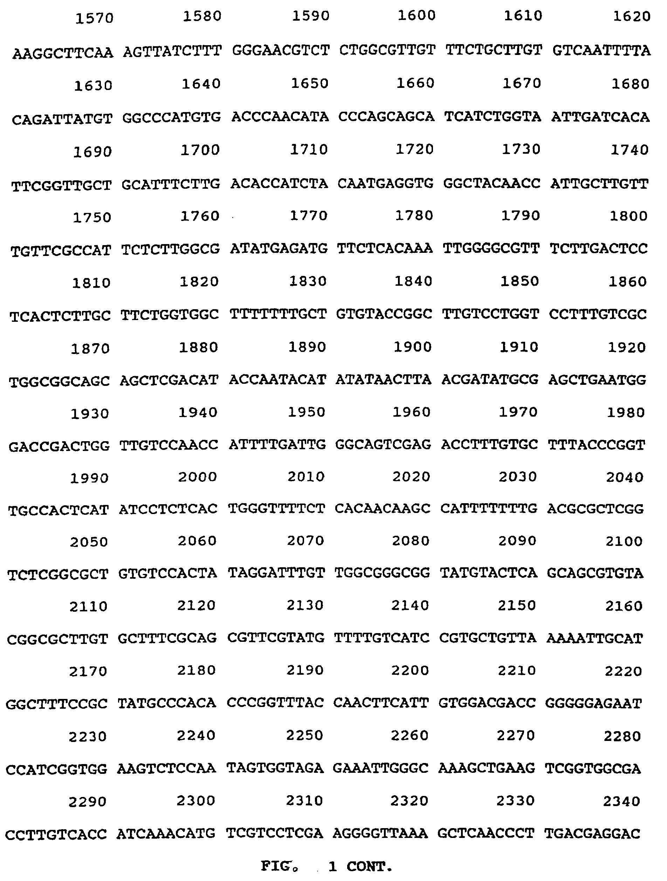

Figure 1, which accompanies this description, shows the complete consecutive sequence of the 3383 bp of the cloned fragment corresponding to the 3' end of the PRRS-Olot viral genome. This nucleotide sequence shows 95% homology in comparison with the corresponding sequences of the LV and TV isolates. These two last isolates show, among themselves, 99% homology. The changes in the nucleotide sequence of the PRRS-Olot isolate are found along the entire sequence, but are concentrated especially in 5' end. We should point out, in comparison with LV, the deletion of three nucleotides at position 1860 of PRRS-Olot.

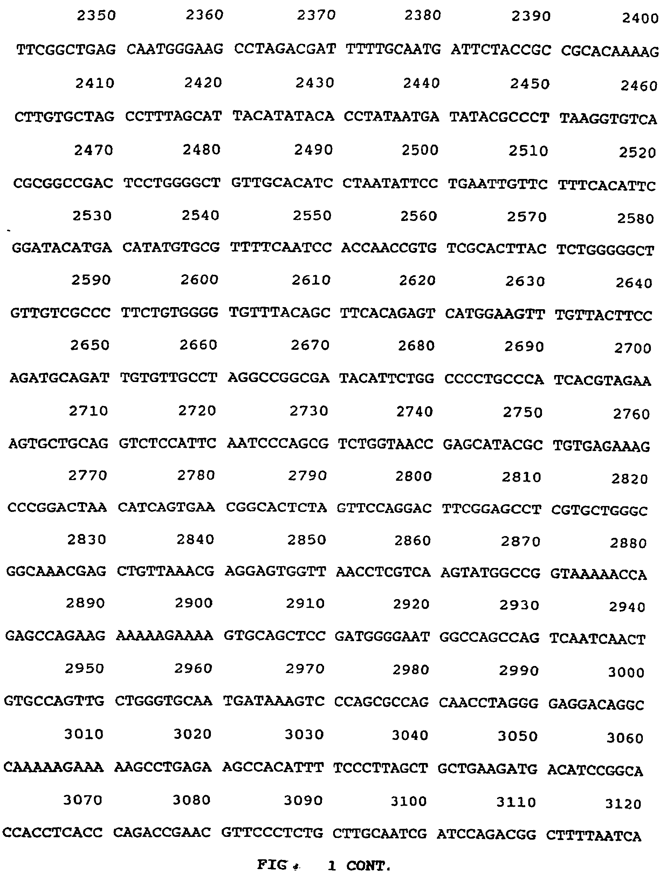

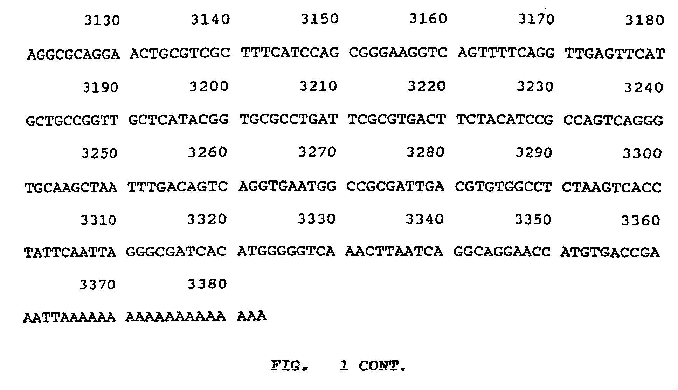

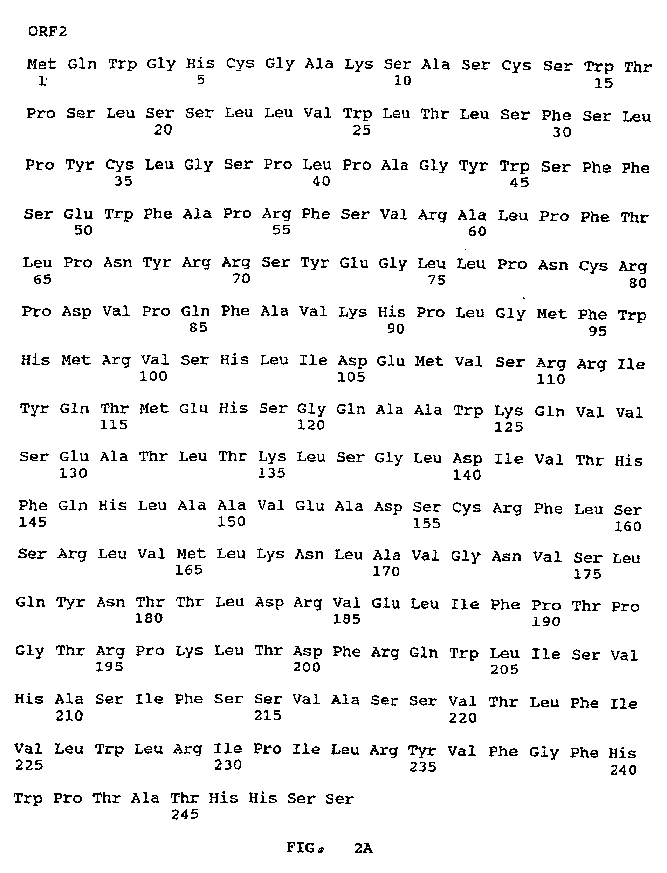

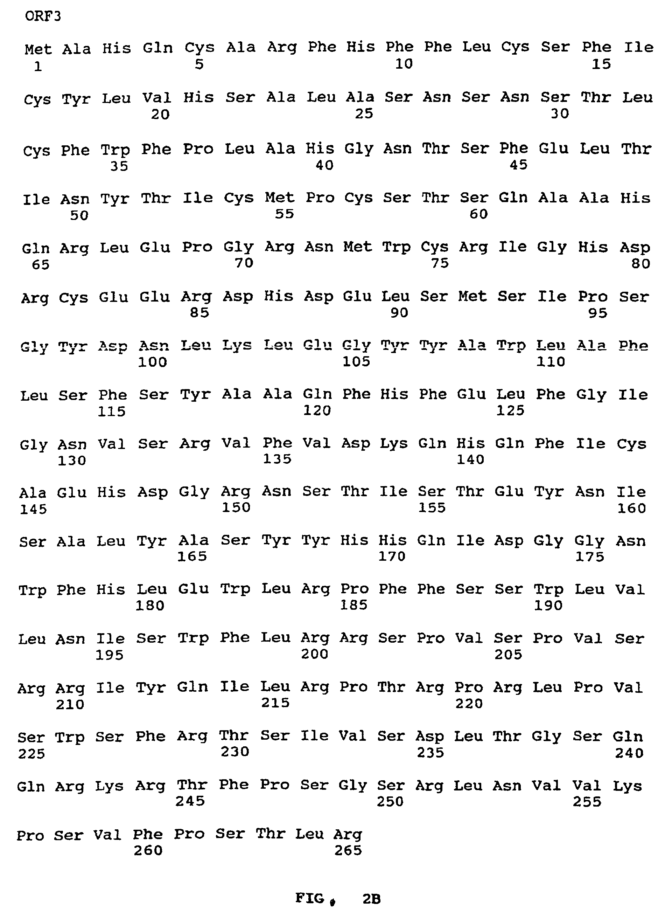

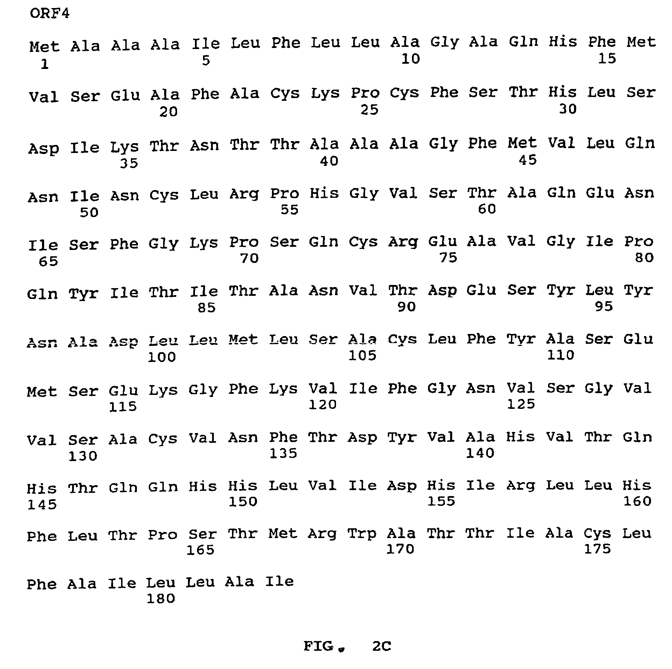

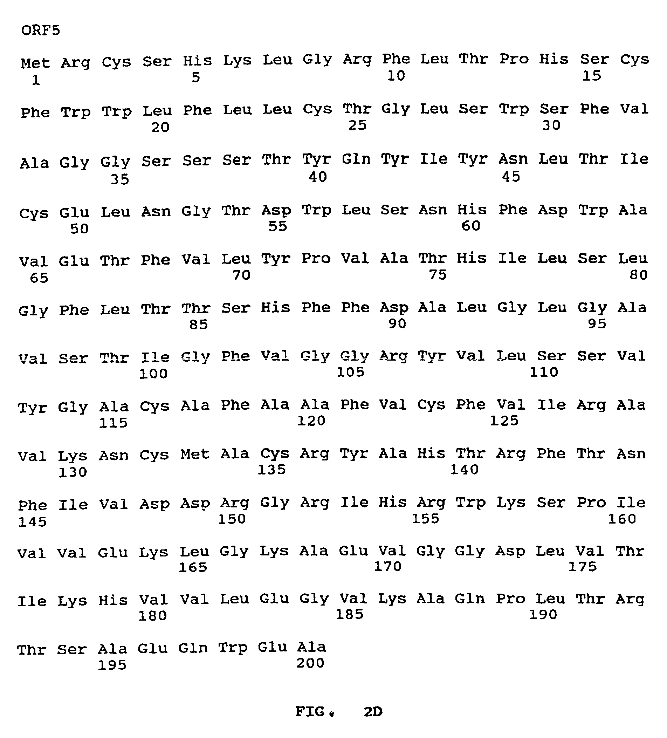

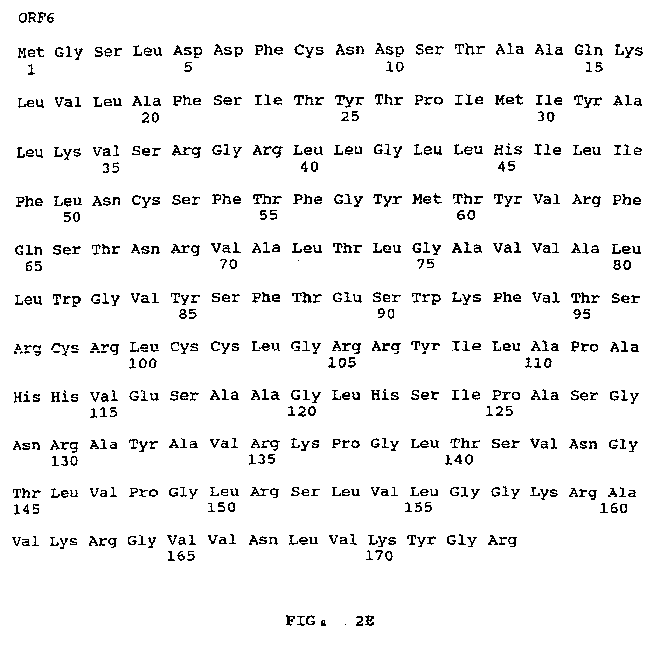

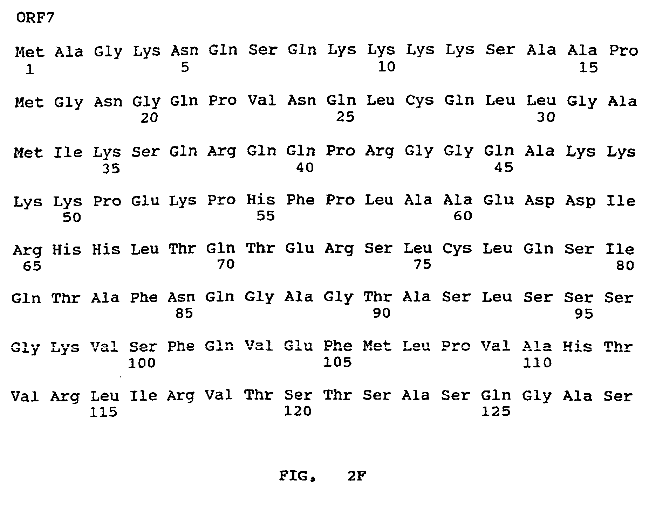

Figure 2 (2A-2F) of this description shows the amino acid sequences of the proteins coded by ORFs 2 to 7 of the PRRS-Olot virus. At protein level, 99% homology is observed between PRRS-Olot and LV ORF7, as expected for a nucleocapsid viral protein and therefore the more conserved. The percentage of homology for the rest of the proteins ranges between 93% for ORFs 3, 4 and 5 reaching a value of 96.5% for ORFs 2 and 6. All of them present glycosilation sites similar to those described for LV except for ORF4 of the PRRS-Olot virus, which has an extra glycosilation site. With regards to the above-mentioned changes in the PRRS-Olot protein amino acids, 50% of the changes are into chemically similar amino acids, whereas the rest of the changes are into different amino acids. As mentioned for LV, excepting ORF7, the rest of the proteins present a high degree of hydrophobicity, possibly in accordance with their association to membranes since they are viral envelope proteins.

Recombinant proteins corresponding to the expression of PRRS-Olot ORFs 2 to 7 can be produced in a suitable expression system and, advantageously, in an expression system of recombinant baculoviruses multiplied in permissive host cell culture. The global procedure for the obtainment of these recombinant proteins basically comprises the following general stages:
I. Preparation of the cDNA sequence to be inserted into a baculovirus; and
II. Obtainment of recombinant baculoviruses expressing the recombinant proteins.

These general stages are in turn subdivided into other sub-stages. This way, the preparation of the cDNA sequence to be inserted comprises the sub-stages of:
I.a Isolation and purification of the PRRS-Olot virus;
I.b Isolation of the viral RNA of the PRRS-Olot virus; and
I.c Synthesizing of the cDNA from the PRRS-Olot genomic RNA.

On the other hand, the obtainment of recombinant baculoviruses expressing the recombinant proteins corresponding to PRRS-Olot ORFs 2 to 7, comprises the sub-stages of:
II.a Preparation of the PRRS-Olot ORF gene to be inserted;
II.b Inserting of the said gene into a baculovirus transfer vector;
II.c Transfection of permissive host cells with the said transfer vector which has the corresponding PRRS-Olot ORF gene inserted.
II.d Selection of the recombinant baculoviruses expressing the recombinant protein corresponding to the inerted ORF.

The characterization of the recombinant baculoviruses and the analysis and purification of the recombinant proteins are then carried out.

All these stages are described in detail further down in this description.

The procedure employed for the obtainment of the recombinant proteins provided by this invention begins with the isolation and purification of the PRRSV, specifically PRRS-Olot, in accordance with the protocol described in Example 1. Once the PRRS-Olot had been isolated and purified, the viral RNA was isolated and for that purpose a commercial kit (Pharmacia) was used, which makes use of a method based on the selection and purification of the viral RNA containing a poly(A) sequence at 3' end (Example 2). The obtained RNA was analyzed in neutral agarose gels at 0.7% by staining with ethidium bromide, and only one band of material with molecular weight of between 5000 and 23000 bp was observed.

Afterwards, the cDNA corresponding to the 3' end viral RNA was synthesized (Example 3) with a commercial kit (Boehringer), by means of a strategy which takes advantage of the presence of a poly(A) tail, and uses an oligo d(T) as extension primer capable of being extended with reverse transcriptase enzyme and synthesize cDNA molecules. To clone the 3' upstream RNA regions, an oligonucleotide annealing to a specific viral genome sequence located approximately at 2500 bp from 3' end was used. A second synthesis was carried out using an oligonucleotide of 20 nucleotides instead of the oligo d(T)₁₂ (Example 3.1). cDNA synthesis was verified and quantitated by means of counting the radioactivity incorporated in the synthesized material and electrophoresis in alkaline and neutral agarose gels. After this, the cloning and sequencing of the cDNA were carried out (Exmple 3.2). To this end, the first thing done was a size selection of synthesized cDNA fragments of between 1000 and 5000 nt (nucleotides). The purified cDNA was cloned in blunt ends in pMTL25 vector.

The analysis of the PRRSV-positive clones was done by means of plasmid DNA preparations and mapping of the restriction sites, based on the LV sequence. Only 9 out of the 300 analyzed plasmids were positive and contained inserts of between 800 and 2600 bp. The definitive verification of the authenticity of these cDNA clones was done by their direct sequencing, using the dideoxys method applied to double-stranded plasmids.

The majority of the obtained positive PRRS clones contained a common poly(A) end and different 5' ends. The clones were denominated as pPRRS-8, pPRRS-108, pPRRS-121, pPRRS-132, pPRRS-146, pPRRS-147, pPRRS-148 and pPRRS-153. Clone pPRRS-3 was extracted from the second synthesis.

To obtain the recombinant baculoviruses expressing the genes of the proteins coded by PRRSV-Olot ORFs 2 to 7, the following procedure was generally and separately followed: First, the gene from each ORF to be inserted was prepared, except the ORF3 gene which did not require previous preparation. For the preparation of these genes and depending on each particular case, the pMTL25, pMTL24 and pMTL22 plasmids were used before they were transferred into baculovirus transfer vectors. The genes corresponding to ORFs 2 to 7 were obtained from the clones that had been obtained previously. After successive manipulations, they originated new recombinant plasmids. The recombinant plasmids, which contained the genes corresponding to each ORF inserted, were purified following the alkaline lysis technique and were characterized by mapping with restriction endonucleases and sequencing of the insertion regions. The new vectors obtained were denominated as pPRRS-ORFN, where N stands for the number of each ORF (N = 2 to 7).

Then, each ORF gene was cloned into a suitable transfer vector. The transfer vector used was pAcYM1 (Matsuura et al., J. Gen Virol. 68, 1233-50). After successive manipulations, new recombinant plasmids, each one of them containing the inserted ORF gene, were originated. The recombinant plasmids obtained were purified following the alkaline lysis technique and characterized by mapping with restriction endonucleases. The insert ends were sequenced in order to verify correct insert region sequence. The new transfer vectors obtained were analyzed to verify that the inserted genes had the correct orientation for their expression by the AcNPV virus polyhedrin promoter. The transfer vectors obtained were:

| Denomination | ORF |
|---|---|
| pPRRS-Bac8 | 2 |
| pPRRS-Bac2 | 3 |
| pPRRS-Bac9 | 4 |
| pPRRS-Bac3 | 5 |
| pPRRS-Bac5 | 6 |
| pPRRS-Bac7 | 7 |

*Spodoptera frugiperda* cells, Sf 9 clone, were then transfected with mixtures of purified infectious DNA of the AcRP23-lacZ parenteral virus and the corresponding transfer vector. Once this transfection had been done, the recombinant baculoviruses were identified by plaque color phenotype assay after the staining of the viral progeny with X-gal, and then purified.

The recombinant baculoviruses obtained were deposited at the European Collection of Animal Cell Cultures (ECACC), Porton Down, Salisbury, Whiltshire SP4 OJG (U.K.).

Examples 4 to 9 describe in detail the obtainment of recombinant baculoviruses expressing the genes coded by ORFs 2 to 7, respectively.

The PRRS-Olot ORF 2 to 7 recombinant proteins can be used with diagnosis purposes to detect the presence of specific PRRSV antibodies (Example 12), and to detect the presence of antigen (PRRSV) by means of antibodies that specifically identify the PRRSV obtained by immunization of animals with, at least, one recombiant protein corresponding to one of PRRS-Olot ORFs 2 to 7.

Additionally, these proteins can also be used to immunize animals against PRRSV. Therefore, the said proteins can be used to formulate recombinant vaccines capable of effectively protecting swine against infection caused by PRRSV. These vaccines may be active or passive. Active vaccines can be prepared by suspending at least one of the recombinant proteins provided by this invention in an immunologically acceptable diluent and an adjuvant. A passive vaccine can be obtained by immunizing animals with the said proteins and isolating the polyclonal antibodies against the said proteins. After antibody isolation and purification, they can be used in vaccine applications.

In a specific realization of this invention, recombinant vaccines are obtained capable of effectively protecting from the infection caused by PRRSV, comprising the viral antigen (antigenic phase) together with an immunologically acceptable diluent and an adjuvant.

For the preparation of the antigenic phase, insect cells -preferentially Spodoptera frugiperda cells- were infected with the diverse recombinant baculoviruses capable of producing the recombinant proteins corresponding to the PRRSV ORFs 2 to 7, and incubated under conditions suitable for the expression of the said proteins. Immediately afterwards, the cells were collected, washed, resuspended in suitable buffer, and then used in the preparation of the aforesaid recombinant vaccines.

In a specific realization, the antigenic phase is composed of a homogenate of insect cells infected with recombinant baculoviruses expressing a single recombinant PRRSV protein, such as, preferently, ORF3, ORF5 and ORF7 (Example 13). In another specific realization, the antigenic phase is composed of a homogenate of a mixture of insect cells infected with different recombinant baculoviruses expressing, each one of them, a different recombinant PRRSV protein, such as a mixture of insect cells infected with the recombinant baculoviruses expressing, for example, the proteins corresponding to ORF3, ORF5 and ORF7.

In general, vaccines were formulated containing as antigenic phase an amount of about 50x10⁶ insect cells infected with baculoviruses expressing the recombinant protein in question. When the vaccine contains diverse recombinant proteins, the antigenic phase is composed of a quantity of about 50x10⁶ insect cells infected with baculoviruses per the recombinant protein in question, i.e., for a formulation of a vaccine containing the proteins corresponding to ORFs 3, 5 and 7, the antigenic phase is composed of about 50x10⁶ insect cells infected with baculoviruses expressing the ORF3 recombinant protein, 50x10⁶ insect cells infected with baculoviruses expressing the ORF5 recombinant protein, and 50x10⁶ insect cells infected with baculoviruses expressing the recombinant ORF7 protein (Example 13).

Phosphate-buffered saline solutions (PBS) or other similar saline solutions may be used as immunologically acceptable diluents.

As adjuvant, in general, any of the adjuvants habitually used to formulate vaccines may be used, either aqueous - such as aluminum hydroxide, alumina gel suspensions, QuilA- or others, like oily adjuvants, based on mineral oils, glycerides and oleic ether-acid derivatives. In particular, it has been confirmed that an oily adjuvant composed of a mixture of Marcol^{(R)} 52, Simulsol^{(R)} 5100 and Montanide^{(R)} 888, gives very good results. Marcol^{(R)} 52 is a low density mineral oil manufactured by ESSO Española S.A., Simulsol^{(R)} 5100 is a polyethoxy oleate ether commercialized by SEPIC, and Montanide^{(R)} 888 is a high purity anhydromannitol octadecenoate ether commercialized by SEPIC.

The vaccines of this invention can also contain cell response potentiator (CRP) substances, i.e., substances that potentiate helper T cell subpopulations (Th₁and Th₂) such as IL-1 (interleukin-1), IL-2, IL-4, IL-5, IL-6, IL-12, g-IFN (gamma interferon), cell necrosis factor and similar substances which could, in theory, provoke cell immunity in vaccinated animals. These CRP substances could be used in vaccine formulations with aqueous as well as oily adjuvants.

Likewise, other types of adjuvants that modulate and immunostimulate cell response can be used, such as MDP (muramyl dipeptide), ISCOM (Immuno Stimulant Complex) or liposomes.

The vaccines of this invention may be obtained by suspending or mixing the antigenic phase with the immunologically acceptable diluent and the adjuvant. When the adjuvant is oily an emulsion is formed which -in a specific and preferred case- if the adjuvant is a mixture of Marcol 52, Simulsol 5100 and Montanide 888 the vaccine will be a double water/oil/water emulsion, type w/o/w.

In the case that the vaccine will contain CRP substances, these substances may be added both to the antigenic phase and to the adjuvant. Alternatively, if the vaccine does not contain any CRP substances, these can be injected, if so desired, simultaneously in a separate site different from the site of inoculation.

Additionally, these vaccines can contain combinations of different porcine pathogens containing, besides one recombinant PRRSV protein or more, one or more of the pathogens mentioned below, allowing for the preparation of polyvalent vaccines. Among these pathogens, but not limited exclusively to them, are Actinobacillus pleuropneumoniae, Haemophilus parasuis, Porcine parvovirus, Leptospira, Escherichia coli, Erysipelothrix rhusiopathiae, Pasteurella multocida, Bordetella bronchiseptica, Porcine respiratory coronavirus, Rotavirus or against the pathogens causative of Aujeszky's disease, swine influenza and transmissible gastoenteritis.

Safety and efficacy trials with the vaccines of the present invention have evidenced that the said vaccines are safe and at the same time efficacious.

It has been possible to confirm that one dose of 2 ml of a quantity of viral antigen or antigenic phase equal to or higher than 50x10⁶ infected insect cells expressing one or more of the recombinant PRRSV proteins, administered via deep intramuscular route followed by a revaccination with a dose of 2 ml of vaccine, can effectively protect vaccinated animals from the infection caused by PRRSV.

Likewise, it has been possible to verify that some of the vaccines object of the trial -those identified as rPRRS C and rPRRS D- are capable of inducing cellular immunity in vaccinated animals, based on the fact that sows vaccinated and revaccinated with the said vaccines did not present serological at the moment of challenge and, nevertheless, they were protected (Example 14, Tables 4 and 10).

With the purpose of determining and evaluating the efficacy of the prepared recombinant vaccines in the prevention of PRRS in pregnant sows, a trial was designed consisting of the vaccination of pregnant sows with the different vaccines and then submitting them to a discharge test with virulent virus. Based on the obtained results, it has been possible to evaluate the efficacy of the vaccines objective of this trial. In order to evaluate the efficacy of these vaccines, the reproductive results, the number both of piglets alive and dead at different stages of the piglets' life period, as well as the analysis of the serological results in sows and piglets were taken into account (Example 14).

### DETAILED DESCRIPTION OF THE INVENTION (EXAMPLES)

### Example 1. - Obtainment and purification of the PRRS-Olot virus.

### 1.1 - Obtainment of pig's lung aleveolar macrophages

1.1.1 -Animals. 7 to 8 week old pigs, a cross between Belgium Landrace and Large White breeds, were used. The animals, from our own farms, were seronegative to the following diseases: Aujeszky's, porcine parvovirosis, foot-and-mouth, classic swine fever, swine influenza (types H1N1 and H3N2) and transmissible gastroenteritis.
1.1.2 -Isolation of macrophages. The animals were anesthetized by injecting in the jugular vein 0.1 g of sodium thiopental per each 10 kg body weight. Then, they were sacrificed and the lungs extracted, after ligating the trachea below the epiglotis and sectioning above the ligation. The extracted lung was washed externally with PBS. Successive internal washings were done (4 to 5) with a total of 500 ml of PBS supplemented with antibiotics at 1:500 (PEG solution: 1000 IU/ml penicillin, 1 mg/ml streptomycin, and 0.5 mg/ml gentamicin), in order to obtain macrophages. These washings were collected together and centrifuged at 300 g for 15 minutes. The following step was to wash the cells twice with PBS by means of consecutive centrifugation/sedimentation, to finally resuspend in DMEMs medium (DMEM supplemented with non-essential amino acids at 100x, GIBCO), containing sodium pyruvate 1 mM, and antibiotics (1:1000 of PEG).

The cells were counted by staining with trypan blue in

Newbauer chamber. 0.1 ml of 10⁻¹ macrophage suspension was added to 0.4 ml of DMEMs and 0.5 ml of trypan blue solution. In the majority of cases the number of cells obtained ranged between 1 and 1.2 x 10⁹.

Sterility controls were carried out on the macrophage cells by means of seedings in culture media suitable for the detection of bacteria and fungi. Absence of mycoplasma was verified by cytochemical detection with DAPI (4',6-diamidino-2-phenylindole) which selectively attaches to the DNA and forms high specificity DNA-DAPI fluorescent complexes.

### 1.2 - Replication of the virus in pig alveolar macrophages.

Cell culture vials (150 cm²) were used, containing 100 ml of a macrophage suspension (3 x 10⁶ cells/ml) in the DMEMs medium described above, except for the addition of fetal calf serum (FCS) at 5%. The cells were infected with PRRS-Olot virus, isolated by Laboratorios Sobrino and denominated PRRS-JPD-P5-6-91 (ECACC, accession number V93070108). Infection was done at 10⁻³ infection multiplicity, and the infected cells were incubated at 37°C for 24 h. After this period had elapsed, the medium was withdrawn and substituted by fresh DMEMs containing 2% FCS and antibiotics; incubation was continued at 37°C.

The cultures were observed periodically with microscope to determine the cytopathic effect (CPE) produced by the virus on the macrophages. Generally, CPE by 3-4 days of infection was 70-80%. Giant deformed cells appeared. Normally, the titre of these preparations was 10^{6.55} TCID₅₀/ml (tissue culture infectious dose 50 per milliliter). Macrophages infected at 10⁻⁴ multiplicity produced viral yields of one order of magnitude less.

The presence of virus in these cells was determined by the immunoperoxidase in monolayer assay on pig macrophage cells obtained as described in Example 1 (1.1.2).

Briefly, this was done the following way: In 96-well titration plaques, 100 µl of macrophages were infected: with 50 µl of PRRS-OlOT virus replicated on macrophages.

The plaques were incubated for 48 hours at 37°C. Once incubation had been completed, the medium was withdrawn and the plaques washed two times with saline solution (0.1M NaCl). Subsequently, they were fixed with 20% formaldehyde after consecutive incubations at 37°C, -30°C and formaldehyde at 20%. After washing twice with saline solution, 50 µl of a 1:50 dilution of an anti-PRRS serum from a challenged animal. Simultaneous incubations were done with a negative serum from an uninfected animal. Incubation was for 1 hour at 37°C. After withdrawal of the previous solution, they were washed two times with saline solution. Immediately, 0.1 µg of Protein A (Sigma) in 50 µl was added and incubated at 37°C for 1 hour. The assay was developed with AEC (3-amino-9-ethyl-carbazole) dissolved in dimethylformamide in the presence of acetate buffer and oxygenated water. After 15-30 minutes at room temperature in darkness, the plates were observed by microscope. Infected cells appeared stained dark red, in comparison with uninfected cells which were colorless.

### 1.3 - PRRS virus purification.

The virus was purified from PRRSV-infected cell cultures. The culture was clarified by means of centrifugation (20 minutes, 6500 g) . The supernatant was concentrated 10X by using a Millipore-Minitan ultrafiltration system (4.5 pSi, 300 kDa pore-size filter). Then, the virus was sedimented by means of centrifugation (5 h, 20000 g). The supernatant was discarded and the precipitate solubilized with PBS containing 1mM phenylmethylsulfonyl fluoride (PMSF) (Sigma) at 4°C, overnight. The virus was purified in discontinuous sucrose gradient (20-50% w/v in PBS) by means of centrifugation at 95000 g for 3 h. Once the centrifugation had been completed, the band containing the virus was extracted from the gradient; diluted with Tris/EDTA buffer and finally centrifuged overnight at 26000 g for virus sedimentation.

The purified virus was analyzed by means of electrophoresis in polyacrilamide-SDS gels at 12% (Laemmli, U.K., Nature, 227:680, 1970). Total protein was detected by staining with coomassie blue, and immunoblots (Towbin, H., Staehelin, T., and Gordon, J., 1979. Proc. Natl. Acad. Sci. USA, 76: 4350 - 4354). The blots were developed with peroxidase-Protein A (Sigma) conjugate using a covalescent anti-PRRSV serum. It was not possible to observe any specific band related with PRRSV in coomassie-stained gels because of contamination with proteins from the macrophages. However, several viral proteins of molecular weights between 15.5 and 30 KDa were identified by immunoblot. With longer developing times, it was also possible to observe bands of molecular weights over 60 KDa but as these were also detected in uninfected macrophages, it was concluded that they were not PRRS virus-related proteins.

### Example 2. - Isolation of the viral RNA

A commercial Pharmacia P-L Biochemicals kit was used.

The method is based on the selection and purification of the viral RNA containing a 3' end poly(A) tail. The viral capsid rupture was done with guanidinium chloride purification of RNA-poly(A) with an oligo-celullose (dT) matrix.

Briefly, the isolation of the PRRS-Olot virus RNA was carried out the following way: The purified virus sedimented by overnight centrifugation at 40000 g. Afterwarads, the supernatant was discarded and the precipitate solubilized with 0.4 ml of the kit extraction buffer. After adsobrption into the cellulose-d(T) matrix, and consecutive washings with the low and high salt concentration buffers, the RNA-poly (A) was eluted with high ClNa concentration. The RNA was precipitated by adding 1:10 volume of 2.5 M potassium acetate, 0.25 mg/ml glycongen and 2 volumes of ethanol (>2 h. at -20°C). Once this period had elapsed, the RNA was recuperated by centrifugation at 16000 g for 30 minutes After washing the precipitate with ethanol at 75%, it was resuspendend in 20 µl of TE buffer (10 mM Tris-ClH pH=8.0 and 1mM EDTA).

The obtained RNA was analyzed in 0.7% neutral agarose gels by staining with ethidium bromide. A single band of material within 5000 and 23000 bp molecular weight was observed. The absence of low molecular weight material must be pointed out and therefore the possibility of cellular DNA or RNA. However, the amount of material obtained was low - not higher than 100 ng of RNA/250 ml of macrophage culture infected with the virus. This low yield agrees with the low yield of purified virus, as shown by electrophoresis in polyacrilamide gels and electron microscopy (data not shown).

### Example 3. - cDNA synthesis from the PRRS-Olot virus genomic RNA

### 3.1 - Preparation of the cDNA.

The cDNA corresponding to the 3' end RNA of the PRRS-Olot viral isolate was synthesized. The strategy takes advantage of the presence of a poly(A) tail in order to use the oligo d(T) as extension primer that can be extended with reverse transcriptase and can synthetize DNA molecule copies. To clone the RNA regions previous to the 3' end, an oligonucleotide with specific sequence of the viral genome located at approximately 2500 bp of the 3' end was used. cDNA synthesis was carried out using a commercial kit (Boehringer). The procedure, in brief, was: 0.1 µg of PRRS RNA-poly(A), obtained as described in the previous example, was incubated in the presence of 1 mM each dNTPs (dATP, dCTP [5-10 µCi of ³²p-α-dCTP], dGTP and dTTP), 25 units of an RNase inhibitor, 0.8 µg oligo d(T)₁₂, and 40 units of reverse transcriptase in 20 µl final volume. The reaction was incubated at 42°C for 1 h and then the synthesis of the second strand was startaed in the same tube. To that end, buffer, RNasa, and 25 units of *E. coli* DNA polymerase were added. Incubation was for 1 hour at 22°C, and 10 minutes at 65°C. Finally, to generate blunt ends, 4 units of DNA T4 polymerase were added. After 10 minutes at 37°C, reaction was stopped by adding EDTA and sarkosyl. A second cDNA synthesis was done under the same conditions, except for the fact that 5'CGGGCTCGAGCCTTTGGCGA3' oligonucleotide was used instead of oligo d(T)₁₂. In both cases, the mixture was extracted with phenol:chloroform and the material was precipitated with ethanol, as described in the previous example. cDNA synthesis was checked and quantified by means of counting the radioactivity incorporated in the synthesized material, and electrophoresis in alkaline and neutral agarose gels.

### 3.2 - Cloning and sequencing.

First, the synthesized cDNA was size selected to avoid the cloning of excessively small segments. For that purpose, the material from the cDNA synthesis was recovered by centrifugation (30 minutes, 16000 g). The precipitate was vacuum dried, dissolved with Tris/EDTA buffer (TE) pH=8.0, and loaded in 1% agarose gel. The cDNA fragments between 1000 and 5000 bp were recovered from the gel with DEAE-cellulose paper and from the latter by elution with ClNa and subsequent precipitation. Purified cDNA was cloned in blunt ends in the pMTL25 vector, a vector derived from the pUC18. With. that purpose, the vector was linearized with *SmaI* and treated with alkaline phosphatase to reduce the vector background. After ligation with DNA T4 ligase, E.coli XL-1Blue competent cells were transformed with the ligation mixture in the presence of X-gal (5-bromo-4-chloro-3-indolyl β-D-galactopyranoside) (Boehringer) and IPTG (Isopropyl β-D-thiogalactopyranoside) (Gold Bioch), which allows the initial selection of recombinant colonies by color (blue colonies without insert in comparison with white ones with insert).

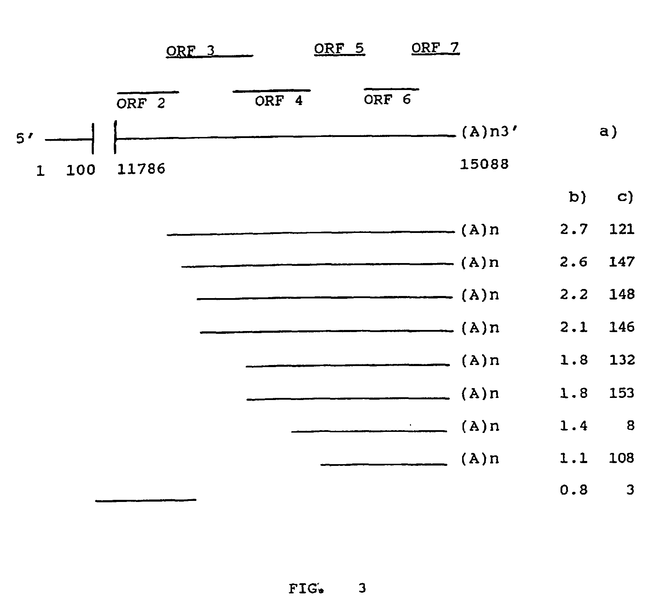

The analysis of the positive PRRS clones was done by means of plasmid DNA preparations (Birnboim & Doly, Nucleic Acids Res., 7, 1513-1523, 1979), and mapping of restriction sites based on LV sequence. Only 9 out of the 300 plasmids analyzed were positive and contained inserts between 800 and 2600 bp. The definitive verification of the authenticity of these cDNA clones was done by their directe sequencing, using the dideoxy chain-termination method applied to double-strand DNA (Sanger, F., et al., J. Mol. Biol., 94:441-448, 1975). The universal oligonucleotides (5'GT_AAAACGACGGCCAGT3') and reverse (5'AACAGCTATCACCATG3') oligonucleotides were used to sequence all the clones. The majority of the obtained PRRS clones contained one common poly(A) tail and different 5' ends. The clones were denominated pPRRS-8, pPRRS-108, pPRRS-121, pPRRS-132, pPRRS-146, pPRRS-147, pPRRS-148 y pPRRS-153. From the second cDNA synthesis, clone PRRS-3 was obtained. Figure 3 shows the different extension of these clones in comparison with LV, as well as the ORFs contained in each one. On the other hand, Figure 1 shows the consecutive sequence of the 3383 bp cloned from the PRRS-Olot isolate, and Figure 2 (2A-2F) shows the amino acid sequences corresponding to the proteins coded by each ORF.

### Example 4. - Obtainment of recombinat baculoviruses expressing the protein gene coded by ORF2

### 4.1 - Preparation of the ORF2 gene

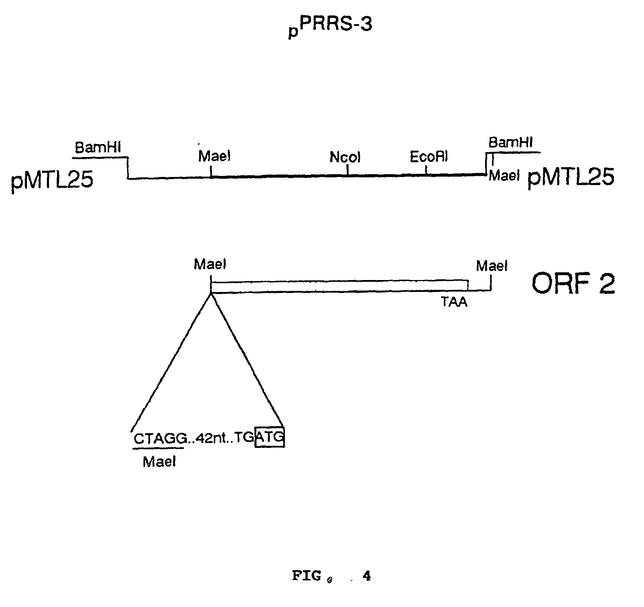

The pMTL25, pMTL24 y pMTL22 genes, derived from the pUC18 vector, were used for the preparation of the different ORFs mentioned in this description, before they were cloned in baculovirus transfer vectors. The vector used is indicated for each particular case. The ORF2 gene is 747 bp in size, and was obtained from cDNA pPRRS-3 clone (Figure 4). The DNA was digested with *MaeI,* and the insert of approximately 900 bp was purified in agarose gel. The cohesive insert ends were transformed into blunt ends by means of treatment with the Klenow fragment of the *E. coli* DNA polymerase. Cloning was done in the pMTL25 treated with *SmaI,* alkaline phosphatase and purified in 1% low melting agarose gel. After ligation with DNA T4 ligase (Boehringer), *E.coli* XL-lBlue cells were transformed with the ligation mixture and the positive clones selected initially by color. The recombinant plasmids containing the inserted ORF2 gene were purified according to the alkaline lysis method (Birnboim & Doly, Nucleic Acids Res., 7, 1513-1523, 1979), and characterized by mapping with restriction endonucleases and sequencing of the insertion regions.

The newly obtained vector was denominated pPRRS-ORF2. In it, the ORF2 initiation codon (ATG) is located approximately at 50 bp from the beginning of the insert and the *BamHI* site.

### 4.2. - Insertion of the ORF2 gene into a baculovirus transfer vector

The baculovirus transfer vector used in all the experiments, described in this patent claim, was pAcYM1 vector (Matsuura et al., J. Gen Virol. 68, 1233-50), which has a single *BamHI* insertion site.

The vector was donated by Professor D.H.L. Bishop (I.V.E.M., Oxford, United Kingdom). For the insertion, the vector was thoroughly digested with the *BamHI* endonuclease and then treated with the alkaline phosphatase enzyme to avoid vector religation. ORF2 codes for a 28.4 KDa protein. Briefly, the insertion of the corresponding gene into the pAcYM1 vector used pPRRS-ORF2 plasmid as a starting material. In this plasmid, the ORF2 gene is flanked by two *BamHI* sites. Thus, the pPRRS-ORF2 is digested with *BamHI* and loaded in 1% low melting agarose gel in order to obtain the 935 bp fragment. This fragment was inserted into the *BamHI* site of pAcYM1 according to Struhl's method (Biotechniques 6, 452-453, 1985), using the DNA T4 ligase (Boehringer) to ligate the insert the vector. The ligation mixture was used to transform *E. coli* DH5 cells. The obtained recombinant plasmids containing the inserted ORF2 gene were purified according to the alkaline lysis method (Birnboin & Doly, supra), characterized by mapping with restriction endonucleases and sequenced the insert edges to corroborate the correct sequence of the insertion regions. The newly obtained transfer vector was denominated pPRRS-Bac8 and it was shown to have the PRRS gene in the correct orientation for its expression by the AcNPV baculovirus polyhedrin promoter.

### 4.3 - Transfection and selection of baculoviruses

*Spodoptera frugiperda* cells, Sf 9 clone, were cotransfected with a mixture of purified infective DNA of parental virus AcRP23-lacZ (500 ng), donated by Dr. Posee (I.V.E.M., Oxford, U.K.) and the transfer vector pPRRS-**Bac8** DNA (2 µg). The parental virus DNA was linearized with the *Bsu36I* enzyme within the *lacZ* gene (Kitts et al., Nuc. Acids Res. 18, 5667-72.1990) in order to increase the efficiency of the recombination. For cotransfection, the lipofectin (Gibco-BRL) method was used (Felgner et al., Proc. Natl. Acad. Sci. U.S.A., 84, 7413-7417, (1987)). After cotransfection, the cells were incubated for 5 days in complete TNMFH medium supplemented with 5% fetal calf serum (FCS) and antibiotics, until cytopathic effect was observed.

Then, the transfection supernatant was recovered and the recombinant viruses identified by plaque assay. The AcRP23-lacZ parental virus shows blue lysis plaques in the presence of X-gal substrate because the β-galactosidase gene is being expressed. Recombinant viruses were initially identified by the clear plaques after staining the viral progeny with X-gal. A number of plaques of each virus were picked and subjected to three purification rounds, before a high titre virus stock was prepared. The recombinant baculovirus finally obtained was denominated AcNPV, PRRS 2. It has been deposited at the European Collection of Animal Cell Cultures (ECACC) with accession number V94021007.

### Example 5 - Obtainment of recombinant baculoviruses expressing the protein gene coded by ORF3

### 5.1 - Insertion of ORF3 gene into a baculovirus transfer vector

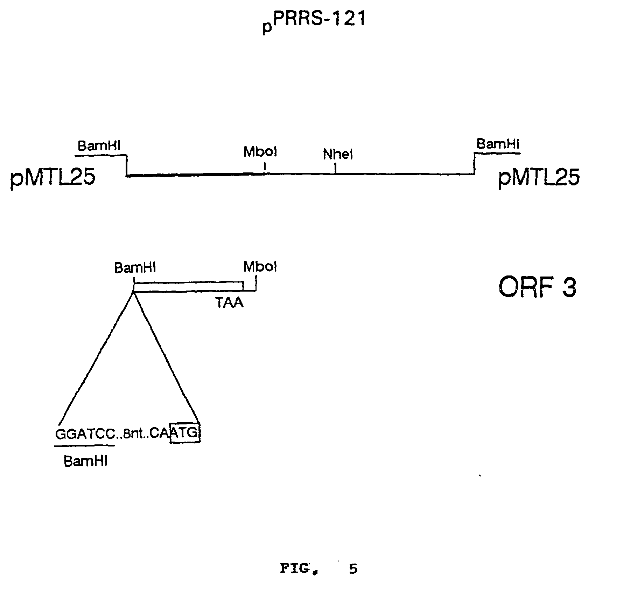

ORF3 codes for a protein of an estimated molecular weight of 30.8 KDa. pPRRS-121 plasmid DNA was used as a starting material for the insertion of the corresponding gene in the pAcYM1 transfer vector (Figure 5). In this vector, the ORF3 initiation codon is located 10 bp from the *BamHI* site. The gene can be excised by double digestion with the *BamHI* and *Sau3A* enzymes, which generates cohesive ends compatible with *BamHI.* After digestion, the mixture was loaded in 1% low melting agarose gel, and a 1009 bp fragment was purified. It was isolated and then ligated to the pAcYM1 vector treated with *BamHI* and alkaline phosphatase, using the T4 ligase DNA enzyme. Subsequently, E. coli DH5 cells were transformed and the recombinant plasmids purified and characterized according to the procedures described above. Once the correct sequence and insert orientation towards the polyhedrin promoter had been verified, the new transfer vector was denominated pPRRS-Bac2.

### 5.2 - Transfection and selection of recombinant baculoviruses

The procedure used for the transfecticn and selection of recombinant baculoviruses was similar to the one described above for ORF2 (Example 4.3). The recombinant baculovirus obtained was denominated AcNPV, PRRS 3. It has been deposited at ECACC with accession number V94011325.

### Example 6. - Obtainment of recombinant baculoviruses expressing the protein gene coded by ORF4

### 6.1 - Preparation of the ORF4 gene

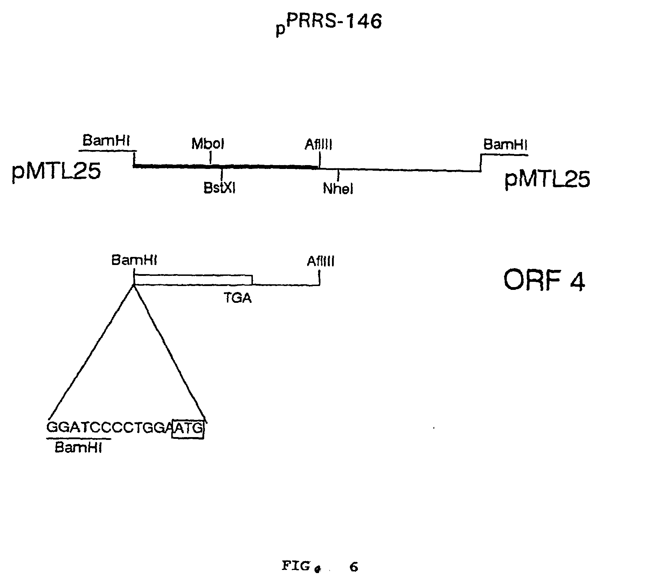

The size of the ORF4 gene is 549 bp. It was obtained from the pPRRS-146 clone (Figure 6) digested with the *BamHI, AflIII* and PstI enzymes. The first two enzymes flank the insert and *PstI* was used to cleave a vector DNA fragment, of similar size to the ORF4 gene which would have made gene isolation difficult. A 1112 bp fragment was purified in low melting agarose gel and cloned in pMTL22 vector digested with *BamHI* and NcoI (compatible with *AflIII*). After ligation with T4 ligase DNA and transformation of E. coli DH5 cells, the recombinant plasmids were purified accoding to the alkaline lysis method (Birmboin & Doly, supra), and characterized by restriction endonuclease mapping. The newly obtained vector was called pPRRS-ORF4. It contains the ORF4 initiation ATG codon located 5 bp from the *BamHI* site.

### 6.2 - Insertion of the ORF4 gene in a baculovirus transfer vector

ORF4 codes for a 20.0 KDa protein. The corresponding gene was obtained from the pPRRS-ORF4 plasmid by digestion with *BamHI* plus *BglII.* The 1112 bp fragment was purified in 1% low melting agarose gel and directly cloned in pAcYMI-*BamHI.* The procedures for the identification and characterization of the recombinant clones were identical to those described above (Example 4.2). Once the correct orientation and insert sequence had been verified, the new plasmid was denominated pPRRS-Bac9. This plasmid was used for posterior transfection experiments and preparation of recombinant baculoviruses.

### 6.3 - Transfection and selection of reombinant baculoviruses

The procedure followed for the transfection and selection of recombinant baculoviruses was similar to the procedure described above for ORF2 (Example 4.3). The recombinant baculovirus was denominated AcNPV, PRRS4. It has been deposited at ECACC with accession number V94021008.

### Example 7. - Obtainment of recombinant baculoviruses expressing the protein gene coded by ORF5

### 7.1 - Preparation of the ORF5 gene

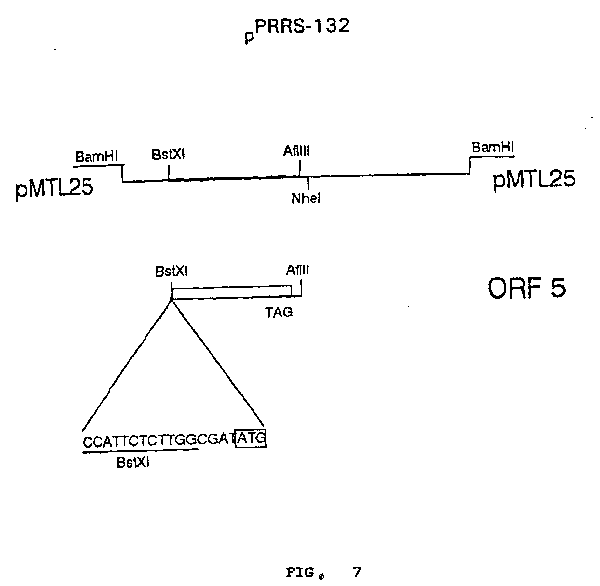

The size of ORF5 is 600 bp. It was obtained from clone pPRRS-132 (Figure 7). The DNA was digested with the *BstXI* and BfrI enzymes, and a 700 bp.fragment containing ORF5 was purified in 1% low melting agarose gel. After converting the fragment ends from cohesive to blunt by means of treatment with T4 polymerase DNA, the fragment was cloned in the pMTL25/SmaI vector. The method used was similar to the procedures described in Example 4.1. The newly obtained vector was denominated pPRRS-ORF5. It contains the ORF5 initiation ATG codon, located 15 bp from the beginning of the gene.

### 7.2 - Insertion of the ORFS gene in a baculovirus transfer vector

ORF5 codes for a 22.4 KDa protein. To insert the corresponding gene in the transfer vector, the pPRRS-ORF5 vector was digested with enzyme *BamHI.* The 706 bp fragment was purified in 1% low melting agarose gel and ligated directly to the pAcYml-BamHI transfer vector. The recombinant plasmids were characterized as described above. The new transfer vector was denominated pPRRS-Bac3. It was used in subsequent transfection experiments.

### 7.3 - Transfection and selection of recombinant baculoviruses

The procedure followed for the transfection and selection of recombinant baculoviruses was similar to the procedure described above for ORF2 (Example 4.3). The recombinant baculovirus obtained was denominated AcNPV, PRRS5 and has been deposited at ECACC with accession number V94011326.

### Example 8. - Obtainment of recombinant baculoviruses expressing the protein gene coded by ORF6

### 8.1 - Preparation of the ORF6 gene

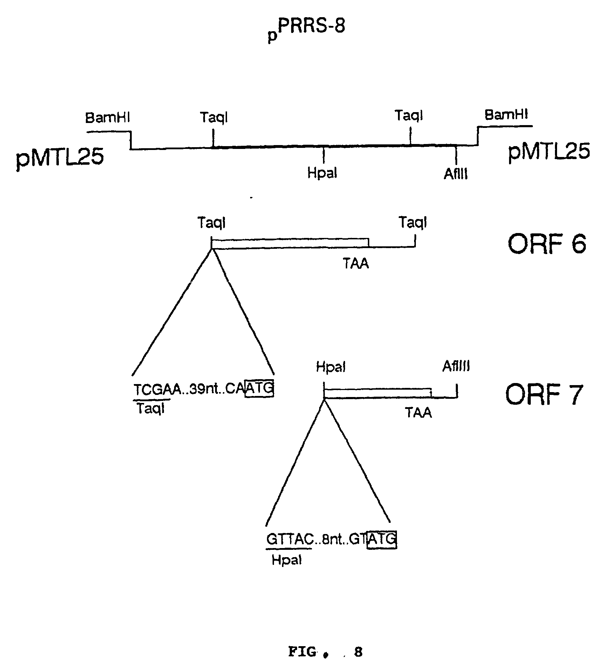

The size of the ORF6 gene is 519 bp. It was prepared from the pPRRS-8 gene clone (Figure 8). First, the DNA was digested with the *AflIII* enzyme, which allowed the elimination of bands approximate in size to the ORF6 gene. A 990 bp *AflIII-AflIII* fragment was purified in 1% low melting agarose gel and digested with TaqI. The new 790 bp fragment was purified in low melting agarose gel and cloned in the pMTL24 vector treated with *AccI* and alkaline phosphatase. Subsequently, the steps described in Example 4.1 were done. The new vector was denominated pPRRS-ORF6. It contains the ORF6 initiation codon located at 46 bp from the beginning of the gene.

### 8.2 - Insertion of the ORF6 gene in a baculovirus transfer vector

ORF6 codes for a 19.0 KDa protein. This is supposed to be the envelope protein and, on account of its hydrophobic nature, it is considered to be a membrane-spanning protein. For the insertion of the corresponding gene in the transfer vector, the pPRRS-ORF6 vector, containing the ORF6 gene cloned at pMTL24 AccI site, was digested with the *BamHI* enzyme. The 790 bp fragment was purified from the 1% agarose gel and ligated directly to vector pAcYM1-*BamHI.* The new transfer vector was denominated **pPRRS-Bac5**. It was used in subsequent transfection experiments.

### 8.3 - Transfection and selection of recombinant baculoviruses

The method used for the transfection and selection of recombinant viruses was similar to the procedure described above for ORF2 (Example 4.3). The recombinant baculovirus obtained was denominated **AcNPV, PRRS6**. It has been deposited at the ECACC with accession number V94011327.

### Example 9. - Obtainment of recombinant baculoviruses expressing the protein gene coded by ORF7

### 9.1 - Preparation of the ORF7 gene

The size of the ORF7 gene is 384 bp. It was prepared from the pPRRS-8 gene clone (Figure 8). Fragment *AfIIII-AfIIII* described in Example 8.1 was digested with the *HpaI* enzyme. The 430 bp *AfIIII-HpaI* fragment containing the ORF7 gene was purified in low melting agarose gel and subsequently cloned in the pPMTL25 vector digested with *NcoI-SmaI.* The analysis and characterization of recombinant colonies was done as described in Example 4.1. The new vector was denominated pPRRS-ORFT. It contains the ORF7 initiaiton codon located at 16 bp from the beginning of the gene.

### 9.2 - Insertion of the ORF7 gene in a baculovirus transfer vector

ORF7 codes for a 13.8 KDa protein. This is supposed to be the viral nucleoprotein. For the insertion of the corresponding gene in the transfer vector, the pPRRS-ORF7 plasmid was digested with the *BglII* and *BamHI* enzymes. The resulting 430 bp fragment was isolated from a low melting agarose gel and ligated directly within the pAcYM1-BamHI vector. After the suitable characterizations, the new pPRRS-Bac7 transfer vector was obtained. It was used in subsequent transfection experiments.

### 9.3 - Transfection and selection of recombinant baculoviruses

The method used for the transfection and selection of recombinant baculoviruses was similar to the procedure described above for ORF2 (Example 4.3). The recombinant baculovirus obtained was denominated AcNPV, PRRS7. It has been deposited at the ECACC with accession number V94011328.

### Example 10. Analysis of recombinant proteins and immunodetection

Sf9 cells were infected with different recombinant baculoviruses at multiplicity of infection of 1 PFU/cell and incubated at 27°C until the cultures were harvested. Different cell cultures were done in monolayer and in suspension. In all the cases, results were similar. The cultures were harvested at different post-infection times. The optimal harvesting time for each recombinant virus was determined. This ranged from between 48 and 96 p.i.h. (post-infection hours). The cells were harvested by centrifugation at 1500 rpm for 10 min, washed twice with PBS pH:7.4 and subsequently resuspend and lysed with 25mM bicarbonate solution. They were centrifuged at 10000 rpm for 10 minutes and the soluble cytoplasmic fraction was separated from the remaining insoluble cell debris. The total cell extracts as well as the different fractions were analyzed by electrophoresis in 11% polyacrilamide gels and stained with coomassie blue or transferred to nitrocellulose membranes for immunological detection. Bands were observed by staining with coomassie blue with molecular weights of 28.4, 30.8, 20.0, 22.4, 19.0 and 13.8 KDa. These sizes correspond respectively to the sizes expected for the genes coded by ORFs 2, 3, 4, 5, 6 and 7. There is a significant variation in the expression levels of the different genes: ORFs 3, 5 and 7 at considerable level, ORFs 2 and 4 at appreciable level and ORF6 at low level. The genes lower expression levels, corresponidng to ORFs 2 and 6, might be due to the larger distance, 42 and 39 nucleotides respectively, between the protein initiation ATG codon and the polyhedrin baculovirus promoter. On several occasions, it has been demonstrated that this distance should essentially be maintained at a minimum in order to obtain a good expression. Another factor, responsible for low expression, could be the high hydrophobic nature of these proteins.

When analyzing separately the soluble and insoluble fractions of the infected cells, it has been observed that, except for ORF7, most of the expressed PRRS proteins are insoluble and remain associated to the membrane debris. This may be due to the hydrophobic and glycosilated nature of these proteins. The majority of these glycoproteins contain transmembrane regions that anchor them to the membranes. Such characteristics make the purification of these proteins from cell extracts difficult.

For immunodetection, the proteins were transferred to nitrocellulose membranes, according to standard methods (Burnette, Anal. Biochem. 112, 195-203, 1981; Towbin et al., Proc. Natl. Acad. Sci. U.S.A. 76, 4350-4354, 1979). Protein transfer was done in a semi-dry device (Bio-Rad) at 22V for 30 minutes. Then, the nitrocellulose strips were blocked with 3% powder skim milk in Tris-HCl 20mM pH 7.5, NaCl 500 mM (TBS) for 1 hour at room temperature. Subsequently, the strips were incubated first for two hours at room temperature with an anti-PRRS pig antiserum (C-45) diluted 1/100 in TBS-0.05% Tween 20, washed with TBS-0.05% Tween 20 for 30 minutes at room temperature, and then incubated with anti-pig IgG conjugated to alkaline phosphatase (dilution 1/1000) (Sigma) for 1 hour. The strips were washed once more and, finally, developed with an NBT (nitro blue tetrazolium) (Sigma) and BCIP (5-bromo-4-chloro-3-indolyl-phosphate) (Sigma) solution in NaCl 100 mM, MgCl₂ 5 mM, diethanolamine 100 mM, pH: 9.5, until the appearance of visible bands. The reaction was stopped with distilled water. In all the cases in which specific reactions were seen by immunoblot, proteins of molecular weight equivalent to the estimated ORF sizes were obtained. In some cases, specifically in ORFs 3 and 5, the presence of other larger-sized bands, till 45 KDa, were observed. These bands would represent different protein glycosilation forms, in agreement with the foreseen potential sites.

### 10.1 - Antigenic characterization of the recombinant proteins

The correct antigenicity of the recombinant proteins expressed in baculovirus was checked by their reaction to different animal sera in an immunoblotting assay.

Recombinant proteins expressed and transferred to nitrocellulose according to the above method, were made to react with a collection of previously characterized swine sera containing anti-PRRSV antibodies. The sera had been obtained in animals infected experimentally (#1-4) or naturally (#5-8).

Proteins corresponding to ORFs 3, 5 and 7 were the first to be checked. Results are shown in Table 2.

**Table 2**

| Reactivity of sera from infected animals against ORF3, ORF5 and ORF7 recombinant proteins | | | |
|---|---|---|---|
| Serum no. | ORF3 | ORF5 | ORF7 |
| 1 | + | + | - |
| 2 | + | + | - |
| 3 | + | + | + |
| 4 | ND | + | + |
| 5 | ND | + | + |
| 6 | + | + | + |
| 7 | ND | + | - |
| 8 | ND | + | + |
| +: Positive - : Negative ND: Not determined | | | |

This assay demonstrated that recombinant proteins 3, 5 and 7 are antigenically similar to native viral proteins 3, 5 and 7, respectively.

When the assay was done with recombinant proteins 2, 4 and 6, the results were of a greater variability in what respects recognition by field sera. The reasons for this variability may be their low expression level and/or their high hydrophobicity.

These assays demonstrate that PRRSV recombinant proteins expressed in baculovirus system are not antigenicallly distinguishable from native viral proteins.

### Example 11. Purification of the recombinant proteins

The strategy designed for recombinant protein purification should take into consideration the structural characteristics of the proteins. Two of these characteristics should be pointed out:
(1) hydrophobic nature which makes them insoluble, and (2) presence of a large number of transmembrane regions which gives them a great affinity to membranes. In most cases, these characteristics do not make protein extraction and purification convenient, e.g.: for their use as a vaccine, when complete infected cells can be used, as described by different authors (Hall S.L., et al., Vaccine, 9, 659-667, Sept. (1991); Tordo N., et al., Virology, 194, 5269 (1993)). In spite of this, some attempts have been made to purify these proteins using ORF3 protein as a model.

### 11.1 - Purification of the protein derived from ORF3

Sf9 cells were infected with the recombinant AcNPV, PRRS3 virus, according to the method described in the previous Example. The infected cells were collected by centrifugation at 400 g for 10 min, washed with PBS and resuspended at 20x10⁶ cells/ml in PBS. The cells were disrupted by freezing/thawing and the soluble fraction was separated from the insoluble fraction by centrifugation. In all the cases, the insoluble fraction was used for the subsequent treatments.

Below is a description of some of the methods used:

### Treatment with chaotropic agents

The insoluble fraction was first washed with 1M NaCl and then with 2M or 4M guanidinium chloride. The cell pellets were resuspended in the different buffers and maintained at room temperature for 1 hour. Then, the preparation was centrifuged at 15000 rpm for 5 minutes The presence of the recombinant protein in the different fractions was analyzed by elecrophoresis in 15% polyacrylamide-SDS gels (sodium dodecyl sodium sulfate).

The results obtained indicate that the sequential treatment with these salts yields a protein of 30% to 50% purity. This purified protein has been shown to be antigenically analogous to native protein, as it is recognizable by sera from infected animals, determined either by immunoblotting or indirect ELISA.

### Treatment with detergents

Detergents at the following concentrations were used:
- NP40 0.5%
- Octylglucoside 2%
- SDS 0.5%, 1% and 2%
- Sodium deoxycholate 0.5%, 1% and 2%

In all cases the cell preparations were done analogous to the one described above. Cell debris containing recombinant protein were treated with the above detergent concentrations and under the described conditions. In general, it can be stated that under these conditions, treatment with the different detergents did not enable the solubilization of a significant amount of recombinant protein. Only 0.5% SDS yielded protein of 50% estimated purity, although with very low yield. Antigenically, this protein reacts with infected animal sera by direct ELISA, although the efficacy is lower than what is obtained with the protein purified with chaotropic agents.

To summarize, these partially purified proteins could be used in anti-PRRSV vaccines.

### Example 12. Diagnostic use

One of the main applications of the recombinant proteins provided by this invention is their use in the preparation of kits for the diagnosis of PRRSV field infections.

### 12.1 - Preparation of antigen expressed in Sf9 for application in diagnosis.

Sf9 cells grown in monolayer or in suspension were infected at multiplicity of infection of 0.5 to 1 with the respective recombinant baculoviruses. Depending on which recombinant virus was used, cultures were harvested between 48 and 72 hours post infection. They were centrifuged at 400 g at 15°C for 10 minutes and washed with PBS.

Finally, the cell pellets containing the recombinant proteins were resuspended in PBS with 2% octylglucoside (Sigma) and were allowed to stand on ice for 1 hour. They were then centrifuged at 1000 g for 10 minutes to eliminate cell debris. The supernatants were exhaustively dialyzed against PBS to remove the detergent, centrifuged at 10000 g for 30 minutes to remove precipitates and stored at -70°C until later use.

### 12.2 - ELISA for diagnosis.

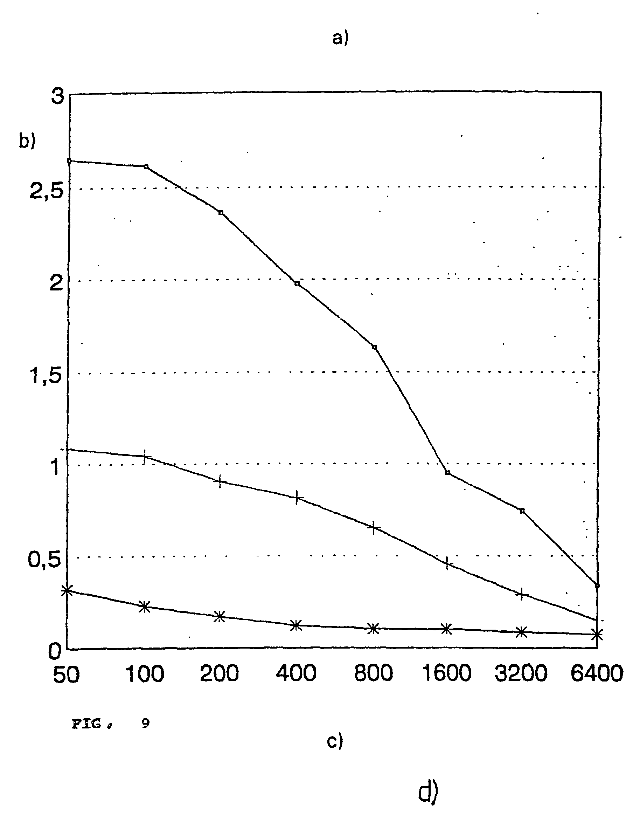

Polystyrene 96-well ELISA immuno plates (Polisorp, NUNC) were coated with different dilutions of the recombinant extracts mixture (ORF2, ORF3, ORF4, ORF5, ORF6 and ORF7), made in 50 mM carbonate buffer pH:9.6 (100 µl/well) by overnight incubation at 4°C. As shown in Figure 9, the optimal dilution chosen for the plate coatings was 1/100. The plates were saturated with blocking buffer (1% skim milk in PBS) for 30 minutes at room temperature. Subsequently, were added different dilutions of the anti-PRRSV antisera made in blocking buffer. Incubation was continued for 1 hour at 37°C. After washing with PBS containing 0.05% Tween 20, peroxidase-labeled protein A (1/5000 dilution) was added, incubating at 37°C for 1 hour. A washing like the previous one was done and the reaction was developed at room temperature for 10 minutes using ABTS [2,2'-azino-bis(3-ethylbenzthiazoline-6 sulfonic acid)] as substrate. The reaction was stopped with 1% SDS and absorbance was monitored at 405 nm.

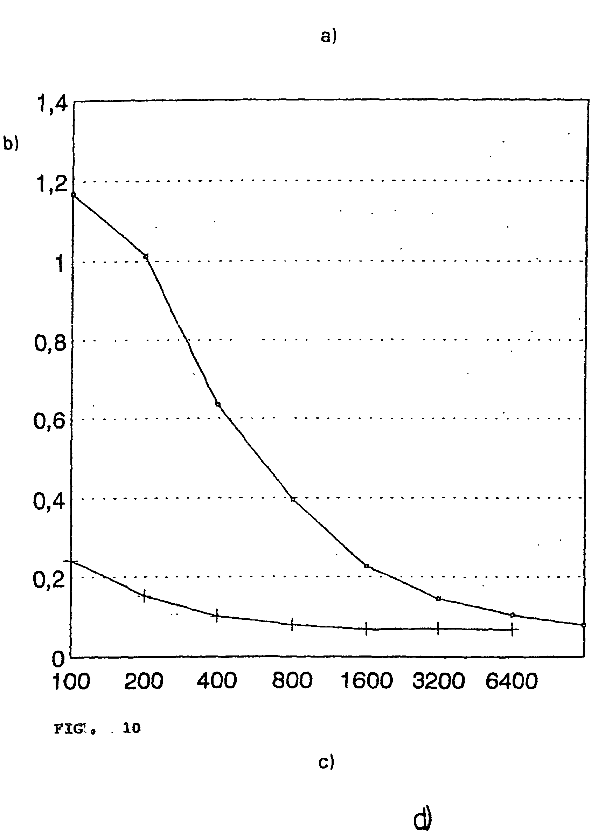

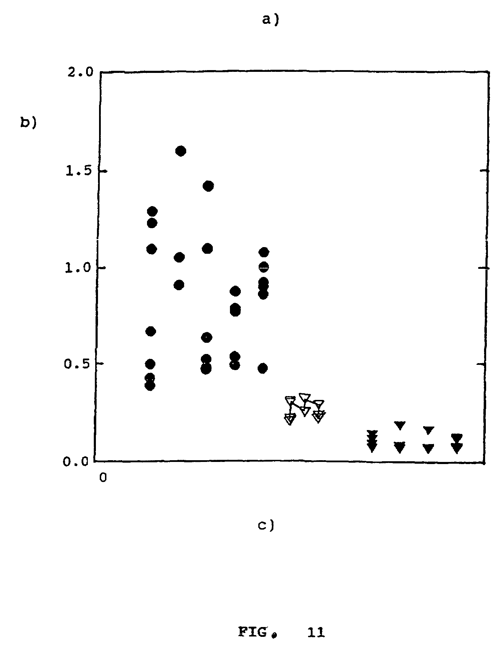

Usual ELISA titration results from an infected animal field serum are shown on Figure 10. Field sera titrations normally range from 1/100 to 1/800 dilutions. The results obtained in a sampling experiment with several dozen field sera are shown on Figure 11. It can be seen that titres obtained for clearly positive sera range from 0.4 to 1.7. Titres from uncertain sera range from 0.2 to 0.3. Negative sera give titres under 0.1. Thus, the conclusion arrived at is: the use of these recombinant proteins expressed in baculovirus is a safe, reliable and reproducible method, which enables to conclusively differentiate infected from uninfected animals.

### Example 13. Formulation of the recombinant vaccines.

Diverse vaccines were prepared containing different recombinant PRRSV proteins, specifically PRRS-Olot [ECACC V93070108] in emulsion form, in accordance with the method described below.

Spodoptera frugiperda cells, clone Sf9 -hereunder Sf9-were infected at the rate of 1x10⁶ cells/ml with the recombinant baculoviruses:
- AcNPV, PRRS3, [ECACC V94011325];
- AcNPV, PRRS5, [ECACC V94011326]; and
- AcNPV, PRRS7, [ECACC V94011328], capable of producing, respectively, the recombinant proteins corresponding to ORF3, ORF5 and ORF7 of the aforesaid PRRSV (Figures 2, 4 and 6), at infection multiplicity of 0.1 plague forming units (PFU)/cell. They were incubated at 27°C, with stirring at 100 rpm and 30% of pO₂, for 72 hours, in a 2 liter Braun-MD fermentor. Then the infected insect cells were collected by centrifuging at 1000 rpm for 10 minutes, washed with phosphate buffered saline solution (PBS) pH:7.4 and suspended at 5x10⁷ cells/ml in the same PBS buffer.

The vaccines were formulated by mixing an infected Sf9 cell homogenate containing 50x10⁶ Sf9 cells expressing each one of recombinant proteins ORF3, ORF5 and ORF7, with an oily adjuvant, or oily phase, composed of a mixture of:

| | |
|---|---|
| Marcol^{(R)} 52 | 790.0 mg |
| Simulsol^{(R)} 5100 | 70.0 mg |
| Montanide^{(R)} 888 | 80.0 mg |

Under these conditions, 4 recombinant vaccines were prepared, in doses of 2 ml, composed of 53% antigenic phase and 47% of the oily phase described above, in which the oily phase/antigenic phase relation is a weight/volume relation (W/V). The prepared vaccines presented the following formulation:
1. Vaccine identified as rPRRS C:
   53%, by volume, of antigenic phase composed of 50x10⁶
   Sf9 cells expressing ORF3; and
   47%, by weight, of the oily phase as described above.
2. Vaccine identifed as rPRRS D:
   53%, by volume, of antigenic phase composed of 50x10⁶
   Sf9 cells expressing ORF5; and
   47%, by weight, of the oily phase as described above.
3. Vaccine identified as rPRRS E:
   53%, by volume, of antigenic phase composed of 50x10⁶
   Sf9 cells expressing ORF7; and
   47%, by weight, of the oily phase as described above.
4. Vaccine identified as rPRRS F:
   53%, by volume, of antigenic phase composed of 50x10⁶
   Sf9 cells expressing ORF3; 50x10⁶ Sf9 cells expressing
   ORF5, and 50x10⁶ Sf9 cells expressing ORF7, (total 150x10⁶ Sf9 cells); and
   47%, by weight, of the oily phase as described above.

### Example 14. Efficacy in pregnant sows

This trial was carried out to evaluate the efficacy of the recombinant vaccines prepared as described in Example 13. To that end, a total of 12 sows -a Landrace X Large White cross- was used. The animals were transferred to the safety stables of the research center.

Two sows were chosen at random (sows no. 400398 and 400298) and were vaccinated with the vaccine identified as rPRRS C. Two sows (sows no. 400118 and 400307) were vaccinated with the vaccine identified as rPRRS D. With the vaccine identified as rPRRS E three sows were vaccinated (sows no. 314010, 313426 and 400059), and with the vaccine identified as rPRRS F three sows were vaccinated (sows no. 313524, 401236 and 401426). The two remaining sows (sows no. 1 and 20) were not vaccinated and were used as control animals.

The sows were vaccinated via deep intramuscular route (IM) in the neck, close to the ear, with a dose of 2ml of vaccine, and revaccinated 21 days later with the same dose.

Local and general reactions were observed, such as: rectal temperature, feed intake and clinical signs both post-vaccination and post-challenge. Additionally, reproductive post-challenge results in the sows were monitored, as well as the serological results both in sows and piglets. The analysis of the results was used in the evaluation of the efficacy of the vaccine (Table 1).

Challenge was done in the safety stables of the research center. All the animal were infected at the rate of 5 ml of PRRSV-218-P6-Mφ-F22055-29/10/94, a strain isolated and maintained at the deposits of the research center, with a titer of 10⁶⁻¹TCID₅₀/ml (tissue culture infectious dose 50%) via intranasal route (IN).

For the evaluation of the sows' reproductive results on the day of farrowing, the following data were noted down (Table 3):
- no. of piglets born alive and in good health
- no. of piglets born alive but weak
- no. of stillborn piglets
- no. of piglets with partial autolysis (edematous)
- no. of mummified piglets
- piglets alive after the 1st week of life, and
- piglets alive at the time of weaning (25-30 days of age).

**Table 3**

| Reproductive results | | | | | | | | | |
|---|---|---|---|---|---|---|---|---|---|
| SOW No. | VACCINE | NUMBER OF PIGLETS | | | | | | | |
| | | TOTAL | BORN ALIVE HEALTHY | BORN ALIVE WEAK | STILL-DORN | PARTIAL AUTOLYS. | MUMMI-FIED | PIGLETS ALIVE 1st WEEK | PIGLETS WEANED |
| 1 | CONTROL | 17 | - | 4 | 9 | 4 | - | - | - |
| 20 | CONTROL | 14 | 9 | - | 2 | 3 | - | 7 | 4 |
| 400398 | rPRRS C | 8 | 8 | - | - | - | - | 7 | 6 |
| 400298 | rPRRS C | 11 | 10 | 1 | - | - | - | 8 | 7 |
| 400118 | rPRRS D | 12 | 6 | 1 | 2 | 3 | - | 5 | - 4 |
| 400307 | rPRRS D | 10 | 9 | - | 1 | - | - | 9 | 7 |
| 314010 | rPRRS E | 12 | - | 10 | 1 | 1 | - | 3 | 2 |
| 313426 | rPRRS E | 6 | 3 | - | - | 1 | 2 | 3 | 3 |
| 400059 | rPRRS E | 12 | 6 | 2 | 2 | 2 | - | 1 | 0 |
| 313524 | rPRRS F | 11 | 10 | - | 1 | - | - | 10 | 8 |
| 401236 | rPRRS F | 2 | - | - | - | - | - | 2 | 2 |
| 401426 | rPRRS F | 15 | 12 | 3 | - | - | - | 10 | 10 |

Then, serological response was analyzed in the sows (Table 4) and piglets (Tables 5, 6, 7, 8 and 9) by means of a peroxidase monolayer assay (IPMA) [Immuno Peroxidase Monolayer Assay, Wensvoort et al., Vet. Quaterly, Vol. 13, nº 3 (July 1991)], in accordance with the following program:
D 0 (Day 0): Bleeding and vaccination
D + 14: Bleeding [at 14 days post-vaccination]
D + 21: Bleeding and revaccination [21 days post-vaccination]
D + 28: Bleeding [28 days post-vaccination]
D + 35: Bleeding [35 days post-vaccination]
D I: Bleeding and challenge
D I+7: Bleeding [at 7 days post-infection]

Serological results in the sows (anti-PRRSV antibodies) are shown in Table 4.

**Table 4**

| Serological results (anti-PRRSV antibodies) | | | | | | | | |
|---|---|---|---|---|---|---|---|---|
| Vaccine | Sow | D 0 | D+14 | D+21 | D+28 | D+35 | D I | D I+7 |
| rPRRS C | 400298 | -- | 320 | 320 | NT | 160 | 320 | ≥640 |
| rPRRS C | 400398 | -- | -- | -- | NT | - | - | ≥640 |
| rPRRS D | 400307 | -- | -- | -- | -- | -- | -- | ≥640 |
| rPRRS D | 400118 | -- | -- | - | - | -- | - | ≥640 |
| rPRRS E | 314010 | -- | ≥640 | ≥640 | ≥640 | ≥640 | 160 | 320-640 |
| rPRRS E | 313426 | - | ≥640 | ≥640 | ≥640 | ≥640 | 320 | ≥640 |
| rPRRS E | 400059 | -- | ≥640 | 320 | NT | ≥640 | ≥640 | ≥640 |
| rPRRS F | 313524 | -- | 320-640 | 320 | ≥640 | ≥640 | 320-640 | ≥640 |
| rPRRS F | 401236 | -- | ≥640 | ≥640 | ≥640 | ≥640 | ≥640 | 320 |
| rPRRS F | 401426 | -- | 320 | NT | NT | 320 | 160 | ≥640 |
| CONTROL | 1 | -- | NT | NT | NT | NT | - | 160 |
| CONTROL | 20 | - | NT | NT | NT | NT | - | 80 |
| [NT: Not tested; --:Negative] | | | | | | | | |

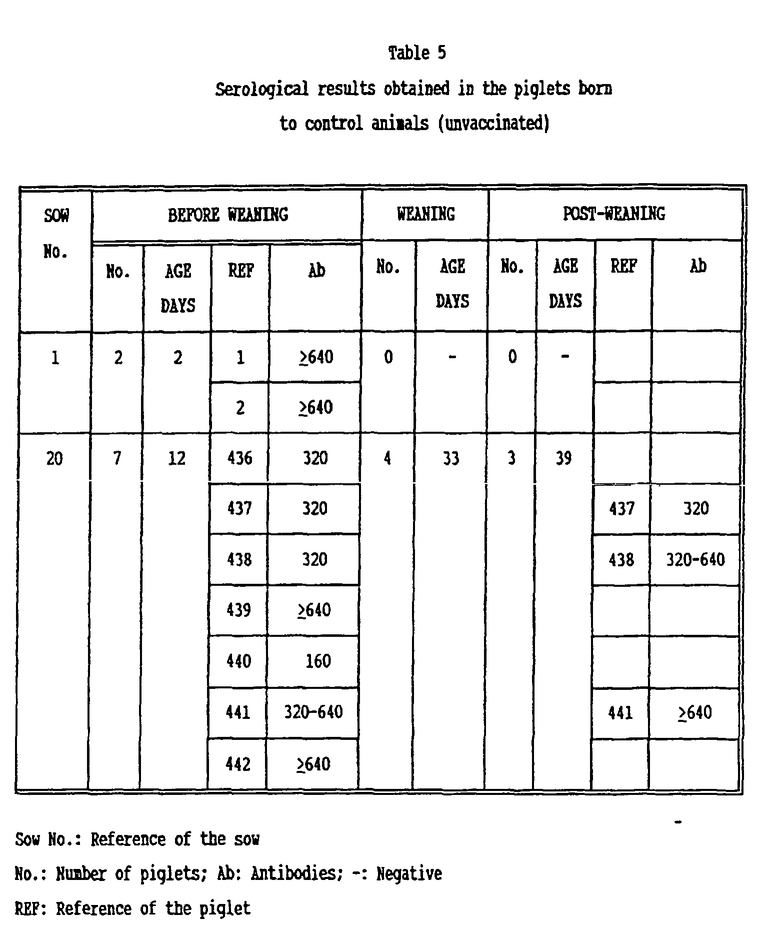

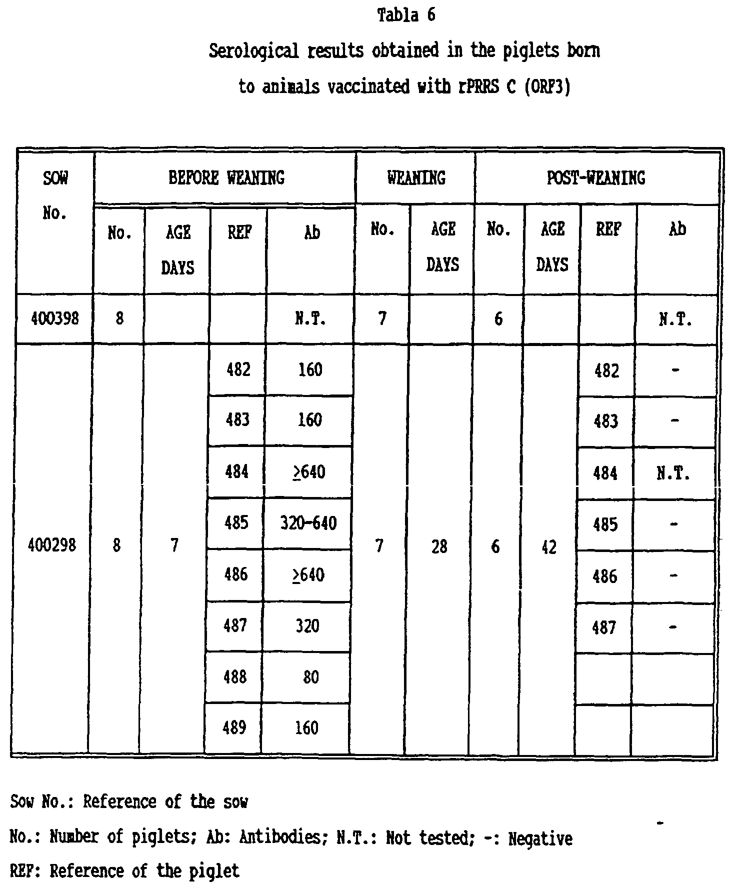

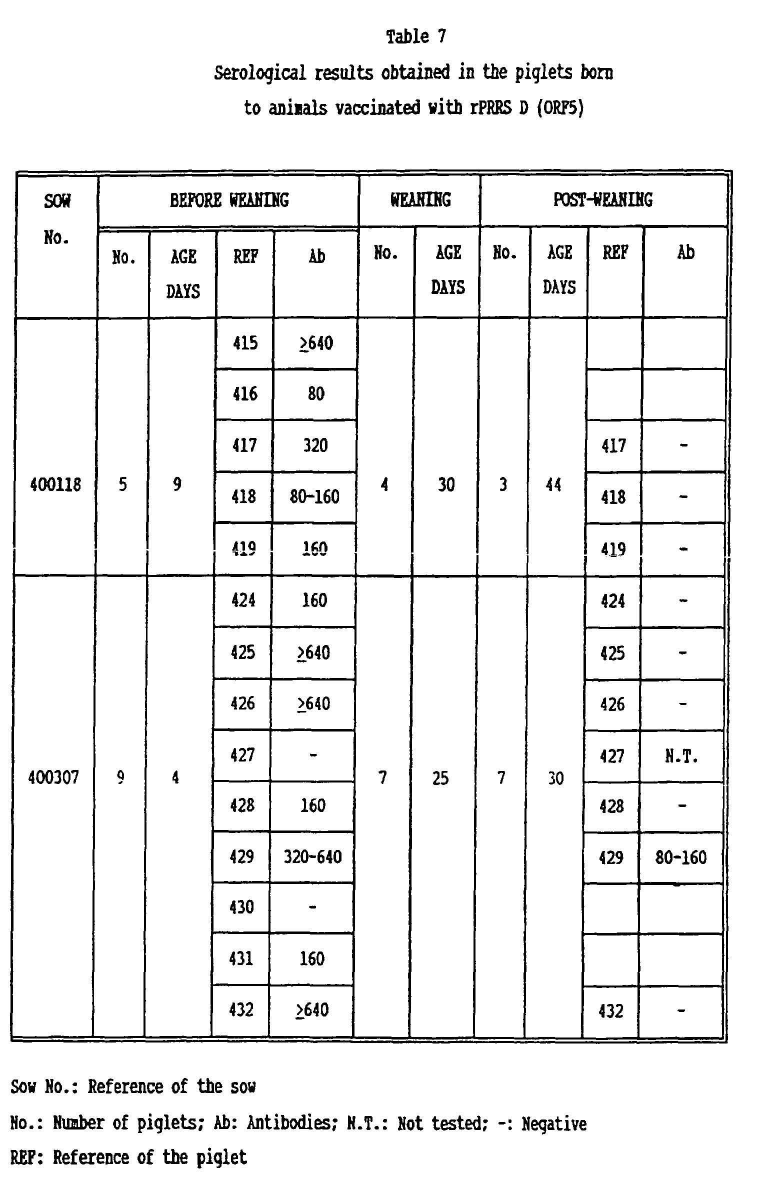

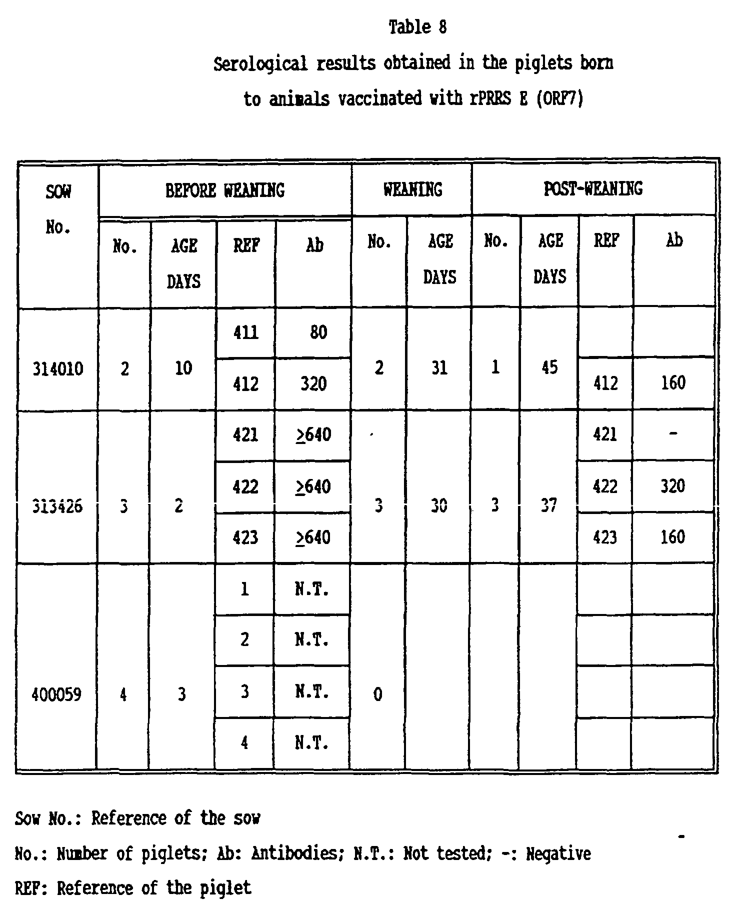

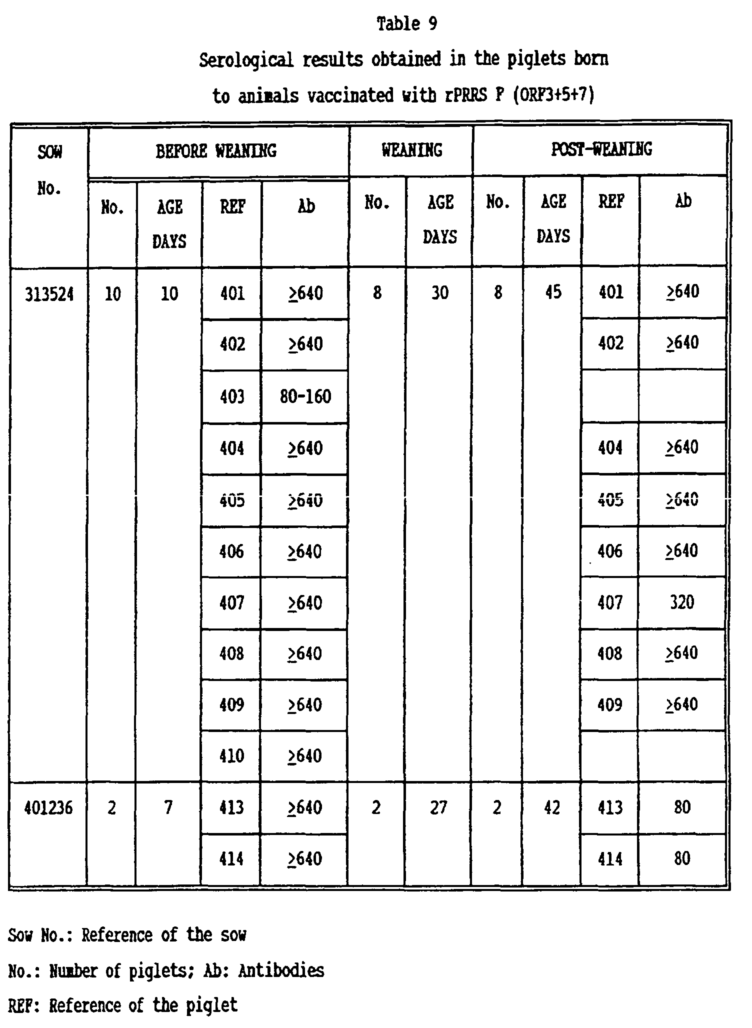

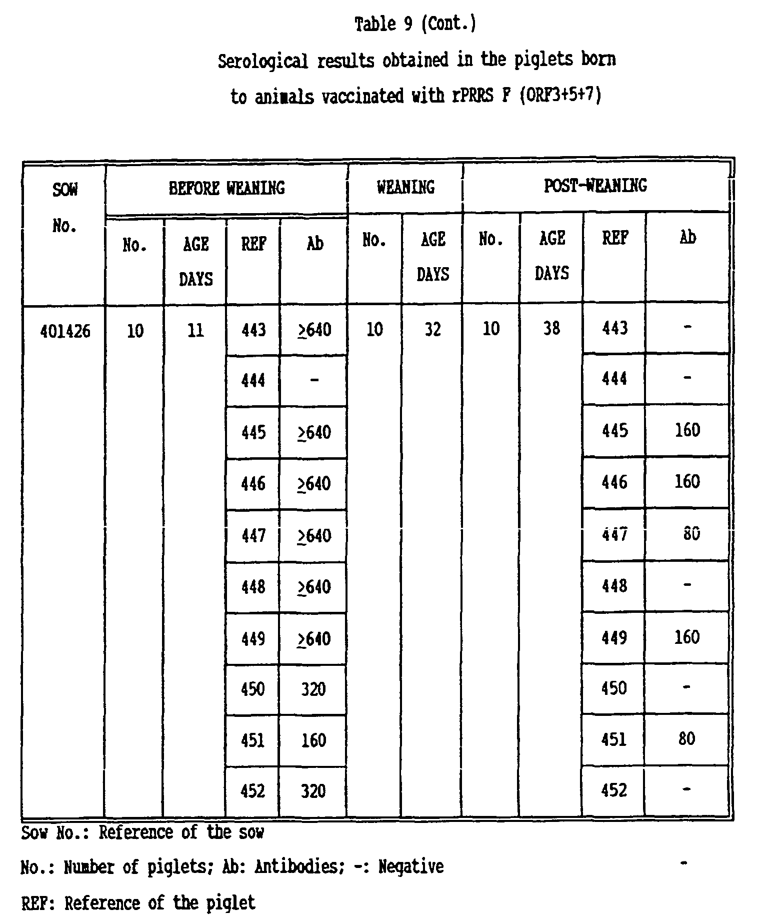

With the purpose of assessing the vaccines object of the trial, serological results as well as reproductive results have been evaluated. Table 10 shows some serological data, while Table 11 summarizes the reproductive data of the sows used in the trials, including information on the total number of piglets born, the number of piglets alive after the 1st week, the number of piglets weaned and the number of piglets of over 40 days of age.

**Table 10**

| Summary of serological and reproductive data | | | | |
|---|---|---|---|---|
| | SOW No. | SEROCONVERSION [IPMA] | | |
| VACCINE | | D O | POST | POST |
| | | | VAC. | INFECTION |
| | | | | (7 days) |
| rPRRS C | 400398 | - | - | + |
| rPRRS C | 400298 | - | + | + |
| rPRRS D | 400118 | - | - | + |
| rPRRS D | 400307 | - | - | + |
| rPRRS E | 314010 | - | + | + |
| rPRRS E | 313426 | - | + | + |
| rPRRS E | 400059 | - | + | + |
| rPRRS F | 313524 | - | + | + |
| rPRRS F | 401236 | - | + | + |
| rPRRS F | 401426 | - | + | + |
| CONTROL | 1 | - | - | + |
| CONTROL | 20 | - | - | + |
| [-: Negative; + : Positive] D O: Time of vaccination | | | | |

**Table 11**

| Summary of reproductive data | | | | | |
|---|---|---|---|---|---|
| VACCINE | SOW No. | N0. OF PIGLETS | | | |
| | | BORN | 1st WEEK | WEANING | > 40 DAYS |
| | 1 | 17 | 0 | 0 | 0 |
| CONTROL | 20 | 14 | 7 | 4 | 3 |
| TOTAL | | 31 | 7 | 4 | 3 |
| rPRRS C ORF3 | 400398 | 8 | 7 | 6 | 6 |
| | 400298 | 11 | 8 | 7 | 6 |
| TOTAL | | 19 | 15 | 13 | 12 |
| rPRRS D ORF5 | 400118 | 12 | 5 | 4 | 3 |
| | 400307 | 10 | 9 | 7 | 7 |
| TOTAL | | 22 | 14 | 11 | 10 |
| rPRRS E ORF 7 | 314010 | 12 | 3 | 2 | 1 |
| | 313426 | 6 | 3 | 3 | 3 |
| | 400059 | 12 | 1 | 0 | 0 |
| TOTAL | | 30 | - | 5 | 4 |
| rPRRS F ORF ORF 3+5+7 | 313524 | 11 | 10 | 8 | 8 |
| | 401236 | 2 | 2 | 2 | 2 |
| | 401426 | 15 | 10 | 10 | 9 |
| TOTAL | | 28 | 22 | 20 | 19 |

The results, in their totality, make it clear that in the case of vaccine rPRRS C, one sow serconverted (400298) and one did not (400398); in the case of vaccine D, none of the sows seroconverted; for vaccines E and F there is strong seroconversion due, chiefly, to the protein coded for ORF 7.

There is a favorable behavior in front of challenge, when the vaccinated animals are compared with those not vaccinated, enabling to assert positively that the recombinant vaccines object of the trial constitute an efficacious means for the prevention of PRRS.

It has been verified that vaccinated sows devoid of antibodies titrated with the IPMA technique are protected, which evidences that the said vaccines (rPRRS C and rPRRS D) are capable of inducing cellular immunity.

The efficacy of the vaccine was evaluated by comparing:
a) The percentage of piglets alive after the 1st week in contrast with the total number of piglets born,
b) the percentage of weaned piglets in contrast with the total number of piglets born, and
c) the percentage of piglets of over 40 days of age in contrast with the total number of piglets born.

Table 12 shows the data relative to the percentage of piglets alive after the 1st week, the percentage of piglets weaned, and the percentage of piglets of over 40 days of age in contrast with the total number of piglets born.

It has been verified that the animals devoid of antibodies, evaluated with the IPMA technique, are protected.

**Table 12**

| Percentage of piglets alive after the 1st week, weaned, and of over 40 days in contrast with the total number of piglets born | | | |
|---|---|---|---|
| VACCINE | % PIGLETS ALIVE 1st WEEK | % PIGLETS WEANED | % PIGLETS >40 DAYS |
| rPRRS C - ORF 3 | 79% | 68.5% | 63% |
| rPRRS D - ORF 5 | 63.6% | 50% | 45.5% |
| rPRRS E - ORF 7 | 23% | 16.6% | 13.3% |
| rPRRS F - ORF 3+5+7 | 78.6% | 71.4% | 67.8% |
| CONTROL | 22.5% | 12.9% | 9.6% |

### DEPOSIT OF MICROORGANISMS

The recombinant baculoviruses obtained were deposited at the European Collection of Animal Cell Cultures (ECACC), Porton Down, Salisbury, Wiltshire SP4 OJG, United Kingdom in accordance with The Budapest Treaty of 1977.

The denomination and accession numbers of the recombinant baculoviruses are:

| Denomination | ECACC Accession Number |
|---|---|
| **AcNPV, PRRS2** | V94021007 |
| **AcNPV, PRRS3** | V94011325 |
| **AcNPV, PRRS4** | V94021008 |
| **AcNPV, PRRS5** | V94011326 |
| **AcNPV, PRRS6** | V94011327 |
| **AcNPV, PRRS7** | V94011328 |

All these baculoviruses were deposited on January 13, 1994, except for AcNPV, PRRS2 (V94021007) and AcNPV, PRRS4 (V94021008) which were deposited on February 10, 1994.

### LEGEND FIGURES

Figure 3:
   a) PRRSV genome
   b) Size (Kb)
   c) Clone number
Figure 9:
   a) Antigen titration by ELISA
   b) Absorbance at 405 nm
   c) Antigen dilutions (1/ )
   d) Serum at 1/200
      -- -- -- Field
      --+--+-- Experimental
      --*--*-- Negative
Figure 10:
   a) Serum titration by ELISA
   b) Absorbance at 405 nm
   c) Serum dilutions (1/ )
   d) Positive -- --- --
      Negative --+--+--
Figure 11:
   a) Field sera titration
   b) Absorbance at 405 nm
   c) Sow sera

## Claims

1. Recombinant proteins of the causative virus of porcine reproductive and respiratory syndrome (PRRS) **characterized** on account of the fact that they are chosen from any of the proteins coded by ORFs 2 to 7 of the virus PRRS-Olot.

2. Proteins as per patent claim 1, **characterized** on account of the fact that they comprise the amino acid sequences shown in Figure 2.

3. Proteins as per patent claim 1, **characterized** on account of the fact that they are obtainable by means of Genetic Engineering in a recombinant baculovirus expression system multiplied in a permissive host cell culture.

4. Proteins as per patent claim 3, **characterized** on account of the fact that such recombinant baculoviruses contain duly inserted and express, at least, the gen of a protein coded by ORFs 2 to 7 of the virus PRRS-Olot.

5. Proteins as per patent claim 3, **characterized** on account of the fact that such permissive host cell culture is a culture of permissive insect cells.

6. Proteins as per patent claim 3, **characterized** on account of the fact that they are obtainable by means of the expression of recombinant baculoviruses chosen from among:
| Denomination | ECACC Accession Number |
|---|---|
| AcNPV, PRRS2 | V94021007 |
| AcNPV, PRRS3 | V94011325 |
| AcNPV, PRRS4 | V94021008 |
| AcNPV, PRRS5 | V94011326 |
| AcNPV, PRRS6 | V94011327 |
| AcNPV, PRRS7 | V94011328 |

7. A procedure for the obtainment of recombinant PRRS-Olot proteins, coded by the genes contained in any of ORFs 2 to 7 of the said virus, which comprises the stages of:
a) preparation of the cDNA sequence, synthesized from the PRRS-Olot genomic RNA, to be inserted in a baculovirus; and
b) obtainment of recombinant baculoviruses that express the recombinant proteins corresponding to the inserted ORFs.

8. A procedure as per patent claim 7, **characterized** on account of the fact that the preparation of the cDNA sequence to be inserted comprises the stages of:
a.1 isolation and purification of the virus PRRS-Olot;
a.2 isolation of the PRRS-Olot viral RNA; and
a.3 synthesis of cDNA from the PRRS-Olot genomic RNA.

9. A procedure as per patent claim 8, **characterized** on account of the fact that the isolation of the virus PRRS-Olot is carried out by replication of the said virus on permissive cell cultures.

10. A procedure as per patent claim 8, **characterized** on account of the fact that the isolation of the PRRS-Olot viral RNA is carried out by adsorption onto an oligo d(T)₁₂-cellulose.

11. A procedure as per patent claim 8, **characterized** on account of the fact that the synthesis of cDNA, from the PRRS-Olot genomic RNA, is carried out by incubating the said RNA with the corresponding dNTPs, reverse transcriptase and either an oligo d(T)₁₂ or, alternatively, an oligonucleotide with formula 5'CGGGCTCGAGCCTTTGGCGA3'.

12. A procedure as per patent claim 8, **characterized** on account of the fact that the obtainment of recombinant baculoviruses expressing recombinant proteins that correspond to ORFs 2 to 7 of PRRS-Olot, comprise the stages of:
b.1 insertion of the corresponding ORF genes in baculovirus transfer vectors;
b.2 transfection of permissive host cells with the said transfer vectors that have inserted the corresponding ORF genes; and
b.3 selection of the recombinant baculoviruses that express the corresponding inserted ORF recombinant proteins.

13. A procedure as per patent claim 12, **characterized** on account of the fact that the said baculovirus transfer vector is vector pAcYM1.

14. A procedure as per patent claim 12, **characterized** on account of the fact that the transfection of the said permissive host cells for the replication of recombinant baculoviruses is carried out with a mixture of DNA of the transfer vector that has inserted the corresponding ORF gene and DNA of the wild-type baculovirus.

15. A procedure as per patent claim 12, **characterized** on account of the fact that the said permissive host cells is an insect cell culture.

16. A procedure as per patent claim 12, **characterized** on account of the fact that the recombinant baculovirus obtained expresses a single recombinant protein of PRRS-Olot, chosen from any of the proteins coded by ORFs 2 to 7 of the said virus.

17. A procedure as per patent claim 12, **characterized** on account of the fact that the recombinant baculoviruses obtained are chosen from among:
| Denomination | ECACC Accession Number |
|---|---|
| AcNPV, PRRS2 | V94021007 |
| AcNPV, PRRS3 | V94011325 |
| AcNPV, PRRS4 | V94021008 |
| AcNPV, PRRS5 | V94011326 |
| AcNPV, PRRS6 | V94011327 |
| AcNPV, PRRS7 | V94011328 |

18. Recombinant baculoviruses, **characterized** on account of the fact that they express, at least, one recombinant protein corresponding to one of ORFs 2 to 7 of PRRS-Olot.

19. Recombinant baculoviruses as per patent claim 18, **characterized** on account of the fact that they express a single recombinant protein of PRRS-Olot, chosen from among any of the proteins coded by ORFs 2 to 7 of the said virus.

20. Recombinant baculoviruses as per patent claim 18, **characterized** on account of the fact they are chosen from among:
| Denomination | ECACC Accession Number |
|---|---|
| AcNPV, PRRS2 | V94021007 |
| AcNPV, PRRS3 | V94011325 |
| AcNPV, PRRS4 | V94021008 |
| AcNPV, PRRS5 | V94011326 |
| AcNPV, PRRS6 | V94011327 |
| AcNPV, PRRS7 | V94011328 |

21. A vaccine suitable for the vaccination and protection of pigs in front of porcine reproductive and respiratory syndrome (PRRS), which comprises, at least, one recombinant protein corresponding to any of the proteins coded by ORFs 2 to 7 of PRRS-Olot and a suitable carrier or adjuvant.

22. A vaccine as per patent claim 21, **characterized** on account of the fact that the said recombinant proteins are obtainable by Genetic Engineering techniques in an expression system of recombinant baculoviruses multiplied in permissive host cell culture.

23. A vaccine as per patent claim 21, **characterized** on account of the fact that the recombinant baculoviruses that express the said recombinant proteins are chosen from among:
| Denomination | ECACC Accession Number |
|---|---|
| AcNPV, PRRS2 | V94021007 |
| AcNPV, PRRS3 | V94011325 |
| AcNPV, PRRS4 | V94021008 |
| AcNPV, PRRS5 | V94011326 |
| AcNPV, PRRS6 | V94011327 |
| AcNPV, PRRS7 | V94011328 |

24. Vaccine as per patent claim 21, **characterized** on account of the fact that said recombinant proteins are used partly purified.

25. Vaccine as per patent claim 21, **characterized** on account of the fact that the said antigenic phase contains a single recombinant PRRSV protein, selected from the group formed by the recombinant proteins coded by PRRS-Olot ORFs 3, 5 and 7.

26. Vaccine as per patent claim 21, **characterized** on account of the fact that the said antigenic phase is composed of insect cells infected with the same recombinant baculovirus expressing only one of the recombinant proteins coded by PRRS-Olot ORFs 2 to 7.

27. Vaccine as per patent claim 21, **characterized** on account of the fact that the said antigenic phase is composed of insect cells infected with different recombinant baculoviruses expressing, each one of them, only one of the different recombinant proteins coded by PRRS-Olot ORFs 2 to 7.

28. Vaccine as per patent claim 21 **characterized** on account of the fact that it contains an oily adjuvant.

29. Vaccine as per patent claim 28, **characterized** on account of the fact that the said oily adiuvant is composed of a mixture of Marcol RTM 52, Simulsol 5100 and Montanide RTM 888.

30. Vaccine as per patent claim 21, **characterized** on account of the fact that it contains an aqueous adjuvant.

31. Vaccine as per patent claim 21, **characterized** on account of the fact that it additionally contains cell response potentiator (CRP) substances such as IL-1, IL-2, IL-4, IL-5, IL-6, IL-12, g-IFN, cell necrosis factor and similar substances.

32. A vaccine as per patent claim 21, **characterized** on account of the fact that the adjuvant is an adjuvant capable of modulating and immunostimulating cell response, such as MDP, ISCOM or liposomes.

33. A vaccine as per patent claim 21, **characterised** on account of the fact that it is capable of inducing cellular immunity in vaccinated animals.

34. A bi- or multivalent vaccine capable of preventing porcine reproductive and respiratory syndrome and another or other porcine infections, **characterized** on account of the fact that it comprises, at least, one recombinant protein corresponding to one of the proteins coded by any of the genes contained in any of ORFs 2 to 7 of PRRS-Olot, together with one or more porcine pathogens and a suitable carrier or adjuvant.

35. A vaccine as per patent claim 343, **characterized** on account of the fact that it includes, at least, one porcine pathogen selected from among the group formed by Actinobacillus pleuropneumoniae, Haemophilus parasuis, Porcine parvovirus, Leptospira, Escherichia coli, Erysipelothrix rhusiopathiae, Pasteurella multocida, Bordetella bronchiseptica, Porcine respiratory coronavirus, Rotavirus or in front of the pathogens causative of Aujeszky's Disease, Swine Influenza or Transmissible Gastroenteritis.

36. A passive vaccine suitable for the vaccination and protection of pigs in front of porcine reproductive and respiratory syndrome (PRRS) **characterized** on account of the fact that it contains antibodies obtained by means of the immunization of animals with, at least, one recombinant protein corresponding to one of the proteins coded by any of the genes contained in any of ORFs 2 to 7 of PRRS-Olot and a suitable carrier or adjuvant.

37. A diagnostic kit for the detection of the presence of antibodies that specifically identify PRRSV in a biological sample, such as blood, serum, sputum, saliva or milk from pigs, comprising, at least, one recombinant protein corresponding to one of ORFs 2 to 7 of PRRS-Olot and suitable detection means.

38. A diagnostic kit as per patent claim 37, **characterized** on account of the fact that the said recombinant proteins are obtainable by Genetic Engineering in an expression system of recombinant baculoviruses multiplied in permissive host cell culture.

39. A diagnostic kit as per patent claim 38, **characterized** on account of the fact that the recombinant baculoviruses expressing the said recombinant proteins are selected from:
| Denomination | ECACC Accession Number |
|---|---|
| **AcNPV, PRRS2** | V94021007 |
| **AcNPV, PRRS3** | V94011325 |
| **AcNPV, PRRS4** | V94021008 |
| **AcNPV, PRRS5** | V94011326 |
| **AcNPV, PRRS6** | V94011327 |
| **AcNPV, PRRS7** | V94011328 |

40. A diagnostic kit for the detection of the presence of PRRSV in a biological sample, such as blood, serum, sputum, saliva, tissue or milk, from pigs, comprising antibodies that specifically identify the PRRSV obtained by immunizing animals with, at least, one recombinant protein corresponding to one of ORFs 2 to 7 of PRRS-Olot and suitable detection means.


SEQUENCE LISTING

(1) GENERAL INFORMATION:

(i) APPLICANT:
(A) NAME: CYANAMID IBERICA S.A.
(B) STREET: CRISTOBAL BORDIU, 35
(C) CITY: MADRID
(D) STATE: MADRID
(E) COUNTRY: SPAIN
(F) POSTAL CODE (ZIP): 28003
(G) TELEPHONE: 34 1 663 91 21
(H) TELEFAX: 34 1 663 94 01

(A) NAME: PLANA DURAN, Juan
(B) STREET: CARRETERA CAMPRODON, S/N, "LA RIBA"
(C) CITY: VALL DE BIANYA
(D) STATE: GERONA
(E) COUNTRY: SPAIN
(F) POSTAL CODE (ZIP): 17813
(G) TELEPHONE: 34 72 19 00 01
(H) TELEFAX: 34 72 29 01 02

(A) NAME: CASAL ALVAREZ, Jose Ignacio
(B) STREET: HERMANOS GARCIA NOBLEJAS, 41
(C) CITY: MADRID
(D) STATE: MADRID
(E) COUNTRY: SPAIN
(F) POSTAL CODE (ZIP): 28037
(G) TELEPHONE: 34 1 368 05 01
(H) TELEFAX: 34 1 408 75 98

(A) NAME: CLIMENT SANCHEZ, Isabel
(B) STREET: CARRETERA CAMPRODON, S/N, "LA RIBA"
(C) CITY: VALL DE BIANYA
(D) STATE: GERONA
(E) COUNTRY: SPAIN
(F) POSTAL CODE (ZIP): 17813
(G) TELEPHONE: 34 72 29 00 01
(H) TELEFAX: 34 72 29 01 02

(ii) TITLE OF INVENTION: PROTEINAS RECOMBINANTES DE PRRSV, KITS DE
DIAGNOSTICO Y VACUNAS QUE CONTIENEN DICHAS PROTEINAS
RECOMBINANTES

(iii) NUMBER OF SEQUENCES: 7

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: WO PCT/ES95/00053
(vi) PRIOR APPLICATION DATA:

(A) APPLICATION NUMBER: ES P9401027
(B) FILING DATE: 13-MAY-1994

(vi) PRIOR APPLICATION DATA:
(A) APPLICATION NUMBER: ES P9500815
(B) FILING DATE: 27-APR-1995

(2) INFORMATION FOR SEQ ID NO: 1:

(i) SEQUENCE CHARACTERISTICS:
(A) LENGTH: 3383 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: NO

(vi) ORIGINAL SOURCE:
(A) ORGANISM: PORCINE REPRODUCTIVE AND RESPIRATORY SYNDROME
VIRUS (PRRSV)
(B) STRAIN: PRRS-OLOT

(vii) IMMEDIATE SOURCE:
(B) CLONE: PRRS-CY-JPD-P5-6-91

(xi) SEQUENCE DESCRIPTION: SEQ ID NO: 1:

GAATTGCAGG TAGAGCTAGG TAAACCCCGG CTGCCGCCTG AGCAAGTGCC GTGAATCCGA 60

AGTGATGCAA TGGGGTCACT GTGGAGCAAA ATCAGCCAGC TGTTCGTGGA CGCCTTCACT 120

GAGTTCCTTG TTAGTGTGGT TGACATTGTC ATTTTCCTTG CCATACTGTT TGGGTTCACC 180

GTTGCCGGCT GGTTACTGGT CTTTCTTCTC AGAGTGGTTT GCTCCGCGCT TCTCCGTTCG 240

CGCTCTGCCA TTCACTCTCC CGAACTATCG AAGGTCCTAT GAAGGCTTGT TACCCAATTG 300

CAGACCGGAT GTCCCACAAT TCGCTGTCAA GCACCCATTG GGTATGTTTT GGCACATGCG 360

AGTCTCCCAC CTAATTGATG AAATGGTCTC TCGTCGCATT TACCAGACCA TGGAACATTC 420

AGGTCAAGCG GCCTGGAAGC AGGTGGTTAG TGAGGCCACT CTTACAAAGC TGTCAGGACT 480

TGATATAGTT ACTCATTTCC AACACCTGGC CGCAGTGGAG GCGGATTCTT GCCGCTTTCT 540

CAGCTCACGA CTTGTGATGC TAAAAAATCT TGCCGTTGGC AATGTGAGCC TACAGTACAA 600

CACCACGTTA GACCGCGTTG AGCTCATCTT CCCTACGCCG GGTACGAGGC CCAAGTTGAC 660

CGATTTCAGA CAATGGCTCA TCAGTGTGCA CGCTTCCATT TTTTCCTCTG TAGCTTCATC 720

TGTTACCTTG TTCATAGTGC TTTGGCTTCG AATTCCAATT CTACGCTATG TTTTTGGTTT 780

CCATTGGCCC ACGGCAACAC ATCATTCGAG CTAACCATCA ACTACACCAT ATGTATGCCC 840

TGCTCTACCA GTCAAGCGGC TCACCAAAGA CTCGAGCCCG GTCGTAACAT GTGGTGCAGA 900

ATAGGGCACG ACAGGTGTGA GGAACGTGAC CATGATGAGT TGTCAATGTC CATTCCGTCT 960

GGGTACGATA ACCTCAAACT TGAGGGTTAT TATGCTTGGC TGGCCTTTTT GTCCTTTTCC 1020

TACGCGGCCC AATTCCATCC GGAGTTGTTC GGAATAGGAA ACGTGTCGCG CGTCTTCGTG 1080

GACAAGCAAC ACCAGTTCAT TTGCGCCGAG CATGATGGAC GAAATTCAAC CATATCTACC 1140

GAATATAACA TCTCCGCATT ATATGCGTCG TACTACCATC ACCAAATAGA CGGGGGCAAC 1200

TGGTTCCATT TGGAATGGCT GCGGCCATTC TTTTCCTCCT GGCTGGTGCT CAACATTTCA 1260

TGGTTTCTGA GGCGTTCGCC TGTAAGCCCT GTTTCTCGAC GCATCTATCA GATATTAAGA 1320

CCAACACGAC CGCGGCTGCC GGTTTCATGG TCCTTCAGAA CATCAATTGT CTCCGACCTC 1380

ACGGGGTCTC AACAGCGCAA GAGAACATTT CCTTCGGGAA GCCGTCTCAA TGTCGTGAAG 1440

CCGTCGGTAT TCCCCAGTAC ATTACGATAA CGGCTAATGT GACCGATGAA TCGTATTTGT 1500

ACAACGCGGA CTTGCTGATG CTTTCTGCGT GCCTTTTCTA CGCTTCAGAA ATGAGCGAAA 1560

AAGGCTTCAA AGTTATCTTT GGGAACGTCT CTGGCGTTGT TTCTGCTTGT GTCAATTTTA 1620

CAGATTATGT GGCCCATGTG ACCCAACATA CCCAGCAGCA TCATCTGGTA ATTGATCACA 1680

TTCGGTTGCT GCATTTCTTG ACACCATCTA CAATGAGGTG GGCTACAACC ATTGCTTGTT 1740

TGTTCGCCAT TCTCTTGGCG ATATGAGATG TTCTCACAAA TTGGGGCGTT TCTTGACTCC 1800

TCACTCTTGC TTCTGGTGGC TTTTTTTGCT GTGTACCGGC TTGTCCTGGT CCTTTGTCGC 1860

TGGCGGCAGC AGCTCGACAT ACCAATACAT ATATAACTTA ACGATATGCG AGCTGAATGG 1920

GACCGACTGG TTGTCCAACC ATTTTGATTG GGCAGTCGAG ACCTTTGTGC TTTACCCGGT 1980

TGCCACTCAT ATCCTCTCAC TGGGTTTTCT CACAACAAGC CATTTTTTTG ACGCGCTCGG 2040

TCTCGGCGCT GTGTCCACTA TAGGATTTGT TGGCGGGCGG TATGTACTCA GCAGCGTGTA 2100

CGGCGCTTGT GCTTTCGCAG CGTTCGTATG TTTTGTCATC CGTGCTGTTA AAAATTGCAT 2160

GGCTTTCCGC TATGCCCACA CCCGGTTTAC CAACTTCATT GTGGACGACC GGGGGAGAAT 2220

CCATCGGTGG AAGTCTCCAA TAGTGGTAGA GAAATTGGGC AAAGCTGAAG TCGGTGGCGA 2280

CCTTGTCACC ATCAAACATG TCGTCCTCGA AGGGGTTAAA GCTCAACCCT TGACGAGGAC 2340

TTCGGCTGAG CAATGGGAAG CCTAGACGAT TTTTGCAATG ATTCTACCGC CGCACAAAAG 2400

CTTGTGCTAG CCTTTAGCAT TACATATACA CCTATAATGA TATACGCCCT TAAGGTGTCA 2460

CGCGGCCGAC TCCTGGGGCT GTTGCACATC CTAATATTCC TGAATTGTTC TTTCACATTC 2520

GGATACATGA CATATGTGCG TTTTCAATCC ACCAACCGTG TCGCACTTAC TCTGGGGGCT 2580

GTTGTCGCCC TTCTGTGGGG TGTTTACAGC TTCACAGAGT CATGGAAGTT TGTTACTTCC 2640

AGATGCAGAT TGTGTTGCCT AGGCCGGCGA TACATTCTGG CCCCTGCCCA TCACGTAGAA 2700

AGTGCTGCAG GTCTCCATTC AATCCCAGCG TCTGGTAACC GAGCATACGC TGTGAGAAAG 2760

CCCGGACTAA CATCAGTGAA CGGCACTCTA GTTCCAGGAC TTCGGAGCCT CGTGCTGGGC 2820

GGCAAACGAG CTGTTAAACG AGGAGTGGTT AACCTCGTCA AGTATGGCCG GTAAAAACCA 2880

GAGCCAGAAG AAAAAGAAAA GTGCAGCTCC GATGGGGAAT GGCCAGCCAG TCAATCAACT 2940

GTGCCAGTTG CTGGGTGCAA TGATAAAGTC CCAGCGCCAG CAACCTAGGG GAGGACAGGC 3000

CAAAAAGAAA AAGCCTGAGA AGCCACATTT TCCCTTAGCT GCTGAAGATG ACATCCGGCA 3060

CCACCTCACC CAGACCGAAC GTTCCCTCTG CTTGCAATCG ATCCAGACGG CTTTTAATCA 3120

AGGCGCAGGA ACTGCGTCGC TTTCATCCAG CGGGAAGGTC AGTTTTCAGG TTGAGTTCAT 3180

GCTGCCGGTT GCTCATACGG TGCGCCTGAT TCGCGTGACT TCTACATCCG CCAGTCAGGG 3240

TGCAAGCTAA TTTGACAGTC AGGTGAATGG CCGCGATTGA CGTGTGGCCT CTAAGTCACC 3300

TATTCAATTA GGGCGATCAC ATGGGGGTCA AACTTAATCA GGCAGGAACC ATGTGACCGA 3360

AATTAAAAAA AAAAAAAAAA AAA 3383

(2) INFORMATION FOR SEQ ID NO: 2:

(i) SEQUENCE CHARACTERISTICS:
(A) LENGTH: 249 amino acids
(B) TYPE: amino acid
(C) STRANDEDNESS: single
(D) TOPOLOGY: linear

(ii) MOLECULE TYPE: protein

(vi) ORIGINAL SOURCE:
(A) ORGANISM: PORCINE REPRODUCTIVE AND RESPIRATORY SYNDROME
VIRUS (PRRSV)
(B) STRAIN: PRRS-OLOT

(vii) IMMEDIATE SOURCE:
(B) CLONE: PRRS-CY-JPD-P5-6-91

(xi) SEQUENCE DESCRIPTION: SEQ ID NO: 2:

Met Gln Trp Gly His Cys Gly Ala Lys Ser Ala Ser Cys Ser Trp Thr

1               5                   10                  15

Pro Ser Leu Ser Ser Leu Leu Val Trp Leu Thr Leu Ser Phe Ser Leu
            20                  25                  30

Pro Tyr Cys Leu Gly Ser Pro Leu Pro Ala Gly Tyr Trp Ser Phe Phe
        35                  40                  45

Ser Glu Trp Phe Ala Pro Arg Phe Ser Val Arg Ala Leu Pro Phe Thr
    50                  55                  60

Leu Pro Asn Tyr Arg Arg Ser Tyr Glu Gly Leu Leu Pro Asn Cys Arg
65                  70                  75                  80

Pro Asp Val Pro Gln Phe Ala Val Lys His Pro Leu Gly Met Phe Trp
                85                  90                  95

His Met Arg Val Ser His Leu Ile Asp Glu Met Val Ser Arg Arg Ile
            100                 105                 110

Tyr Gln Thr Met Glu His Ser Gly Gln Ala Ala Trp Lys Gln Val Val
        115                 120                 125

Ser Glu Ala Thr Leu Thr Lys Leu Ser Gly Leu Asp Ile Val Thr His
    130                 135                 140

Phe Gln His Leu Ala Ala Val Glu Ala Asp Ser Cys Arg Phe Leu Ser
145                 150                 155                 160

Ser Arg Leu Val Met Leu Lys Asn Leu Ala Val Gly Asn Val Ser Leu
                165                 170                 175

Gln Tyr Asn Thr Thr Leu Asp Arg Val Glu Leu Ile Phe Pro Thr Pro
            180                 185                 190

Gly Thr Arg Pro Lys Leu Thr Asp Phe Arg Gln Trp Leu Ile Ser Val
        195                 200                 205

His Ala Ser Ile Phe Ser Ser Val Ala Ser Ser Val Thr Leu Phe Ile
    210                 215                 220

Val Leu Trp Leu Arg Ile Pro Ile Leu Arg Tyr Val Phe Gly Phe His
225                 230                 235                 240

Trp Pro Thr Ala Thr His His Ser Ser
                245

(2) INFORMATION FOR SEQ ID NO: 3:

     (i) SEQUENCE CHARACTERISTICS:
          (A) LENGTH: 265 amino acids
          (B) TYPE: amino acid
          (C) STRANDEDNESS: single
          (D) TOPOLOGY: linear

    (ii) MOLECULE TYPE: protein

(vi) ORIGINAL SOURCE:
(A) ORGANISM: PORCINE REPRODUCTIVE AND RESPIRATORY SYNDROME
VIRUS (PRRSV)
(B) STRAIN: PRRS-OLOT

(vii) IMMEDIATE SOURCE:
(B) CLONE: PRRS-CY-JPD-P5-6-91

(xi) SEQUENCE DESCRIPTION: SEQ ID NO: 3:

Met Ala His Gln Cys Ala Arg Phe His Phe Phe Leu Cys Ser Phe Ile
1               5                   10                  15

Cys Tyr Leu Val His Ser Ala Leu Ala Ser Asn Ser Asn Ser Thr Leu
            20                  25                  30

Cys Phe Trp Phe Pro Leu Ala His Gly Asn Thr Ser Phe Glu Leu Thr
        35                  40                  45

Ile Asn Tyr Thr Ile Cys Met Pro Cys Ser Thr Ser Gln Ala Ala His
    50                  55                  60

Gln Arg Leu Glu Pro Gly Arg Asn Met Trp Cys Arg Ile Gly His Asp
65                  70                  75                  80

Arg Cys Glu Glu Arg Asp His Asp Glu Leu Ser Met Ser Ile Pro Ser
                85                  90                  95

Gly Tyr Asp Asn Leu Lys Leu Glu Gly Tyr Tyr Ala Trp Leu Ala Phe
            100                 105                 110

Leu Ser Phe Ser Tyr Ala Ala Gln Phe His Phe Glu Leu Phe Gly Ile
        115                 120                 125

Gly Asn Val Ser Arg Val Phe Val Asp Lys Gln His Gln Phe Ile Cys
    130                 135                 140

Ala Glu His Asp Gly Arg Asn Ser Thr Ile Ser Thr Glu Tyr Asn Ile
145                 150                 155                 160

Ser Ala Leu Tyr Ala Ser Tyr Tyr His His Gln Ile Asp Gly Gly Asn
                165                 170                 175

Trp Phe His Leu Glu Trp Leu Arg Pro Phe Phe Ser Ser Trp Leu Val
            180                 185                 190

Leu Asn Ile Ser Trp Phe Leu Arg Arg Ser Pro Val Ser Pro Val Ser
        195                 200                 205

Arg Arg Ile Tyr Gln Ile Leu Arg Pro Thr Arg Pro Arg Leu Pro Val
    210                 215                 220

Ser Trp Ser Phe Arg Thr Ser Ile Val Ser Asp Leu Thr Gly Ser Gln
225                 230                 235                 240

Gln Arg Lys Arg Thr Phe Pro Ser Gly Ser Arg Leu Asn Val Val Lys
                245                 250                 255

Pro Ser Val Phe Pro Ser Thr Leu Arg
            260                 265

(2) INFORMATION FOR SEQ ID NO: 4:

    (i) SEQUENCE CHARACTERISTICS:
        (A) LENGTH: 183 amino acids
        (B) TYPE: amino acid
        (C) STRANDEDNESS: single
        (D) TOPOLOGY: linear

    (ii) MOLECULE TYPE: protein

    (vi) ORIGINAL SOURCE:
        (A) ORGANISM: PORCINE REPRODUCTIVE AND RESPIRATORY SYNDROME
                VIRUS (PRRSV)
        (B) STRAIN: PRRS-OLOT

    (vii) IMMEDIATE SOURCE:
        (B) CLONE: PRRS-CY-JPD-P5-6-91

    (xi) SEQUENCE DESCRIPTION: SEQ ID NO: 4:

Met Ala Ala Ala Ile Leu Phe Leu Leu Ala Gly Ala Gln His Phe Met
1               5                   10                  15

Val Ser Glu Ala Phe Ala Cys Lys Pro Cys Phe Ser Thr His Leu Ser
            20                  25                  30

Asp Ile Lys Thr Asn Thr Thr Ala Ala Ala Gly Phe Met Val Leu Gln
        35                  40                  45

Asn Ile Asn Cys Leu Arg Pro His Gly Val Ser Thr Ala Gln Glu Asn
    50                  55                  60

Ile Ser Phe Gly Lys Pro Ser Gln Cys Arg Glu Ala Val Gly Ile Pro
65                  70                  75                  80

Gln Tyr Ile Thr Ile Thr Ala Asn Val Thr Asp Glu Ser Tyr Leu Tyr
                85                  90                  95

Asn Ala Asp Leu Leu Met Leu Ser Ala Cys Leu Phe Tyr Ala Ser Glu
            100                 105                 110

Met Ser Glu Lys Gly Phe Lys Val Ile Phe Gly Asn Val Ser Gly Val
        115                 120                 125

Val Ser Ala Cys Val Asn Phe Thr Asp Tyr Val Ala His Val Thr Gln
    130                 135                 140

His Thr Gln Gln His His Leu Val Ile Asp His Ile Arg Leu Leu His
145                 150                 155                 160

Phe Leu Thr Pro Ser Thr Met Arg Trp Ala Thr Thr Ile Ala Cys Leu
                165                 170                 175

Phe Ala Ile Leu Leu Ala Ile
            180

(2) INFORMATION FOR SEQ ID NO: 5:

    (i) SEQUENCE CHARACTERISTICS:
        (A) LENGTH: 200 amino acids
        (B) TYPE: amino acid
        (C) STRANDEDNESS: single
        (D) TOPOLOGY: linear

    (ii) MOLECULE TYPE: protein

    (vi) ORIGINAL SOURCE:
        (A) ORGANISM: PORCINE REPRODUCTIVE AND RESPIRATORY SYNDROME
            VIRUS (PRRSV)
        (B) STRAIN: PRRS-OLOT

    (vii) IMMEDIATE SOURCE:
        (B) CLONE: PRRS-CY-JPD-P5-6-91

    (xi) SEQUENCE DESCRIPTION: SEQ ID NO: 5:

Met Arg Cys Ser His Lys Leu Gly Arg Phe Leu Thr Pro His Ser Cys
1               5                   10                  15

Phe Trp Trp Leu Phe Leu Leu Cys Thr Gly Leu Ser Trp Ser Phe Val
            20                  25                  30

Ala Gly Gly Ser Ser Ser Thr Tyr Gln Tyr Ile Tyr Asn Leu Thr Ile
        35                  40                  45

Cys Glu Leu Asn Gly Thr Asp Trp Leu Ser Asn His Phe Asp Trp Ala
    50                  55                  60

Val Glu Thr Phe Val Leu Tyr Pro Val Ala Thr His Ile Leu Ser Leu
65                  70                  75                  80

Gly Phe Leu Thr Thr Ser His Phe Phe Asp Ala Leu Gly Leu Gly Ala
                85                  90                  95

Val Ser Thr Ile Gly Phe Val Gly Gly Arg Tyr Val Leu Ser Ser Val
            100                 105                 110

Tyr Gly Ala Cys Ala Phe Ala Ala Phe Val Cys Phe Val Ile Arg Ala
        115                 120                 125

Val Lys Asn Cys Met Ala Cys Arg Tyr Ala His Thr Arg Phe Thr Asn
    130                 135                 140

Phe Ile Val Asp Asp Arg Gly Arg Ile His Arg Trp Lys Ser Pro Ile

145                 150                 155                 160

Val Val Glu Lys Leu Gly Lys Ala Glu Val Gly Gly Asp Leu Val Thr
                165                 170                 175

Ile Lys His Val Val Leu Glu Gly Val Lys Ala Gln Pro Leu Thr Arg
            180                 185                 190

Thr Ser Ala Glu Gln Trp Glu Ala
        195                 200

(2) INFORMATION FOR SEQ ID NO: 6:

     (i) SEQUENCE CHARACTERISTICS:
          (A) LENGTH: 173 amino acids
          (B) TYPE: amino acid
          (C) STRANDEDNESS: single
          (D) TOPOLOGY: linear

    (ii) MOLECULE TYPE: protein

    (vi) ORIGINAL SOURCE:
          (A) ORGANISM: PORCINE REPRODUCTIVE AND RESPIRATORY SYNDROME
               VIRUS (PRRSV)
          (B) STRAIN: PRRS-OLOT

   (vii) IMMEDIATE SOURCE:
          (B) CLONE: PRRS-CY-JPD-P5-6-91

    (xi) SEQUENCE DESCRIPTION: SEQ ID NO: 6:

Met Gly Ser Leu Asp Asp Phe Cys Asn Asp Ser Thr Ala Ala Gln Lys
1               5                   10                  15

Leu Val Leu Ala Phe Ser Ile Thr Tyr Thr Pro Ile Met Ile Tyr Ala
            20                  25                  30

Leu Lys Val Ser Arg Gly Arg Leu Leu Gly Leu Leu His Ile Leu Ile
        35                  40                  45

Phe Leu Asn Cys Ser Phe Thr Phe Gly Tyr Met Thr Tyr Val Arg Phe
    50                  55                  60

Gln Ser Thr Asn Arg Val Ala Leu Thr Leu Gly Ala Val Val Ala Leu
65                  70                  75                  80

Leu Trp Gly Val Tyr Ser Phe Thr Glu Ser Trp Lys Phe Val Thr Ser
                85                  90                  95

Arg Cys Arg Leu Cys Cys Leu Gly Arg Arg Tyr Ile Leu Ala Pro Ala
            100                 105                 110

His His Val Glu Ser Ala Ala Gly Leu His Ser Ile Pro Ala Ser Gly
        115                 120                 125

Asn Arg Ala Tyr Ala Val Arg Lys Pro Gly Leu Thr Ser Val Asn Gly
    130                 135                 140

Thr Leu Val Pro Gly Leu Arg Ser Leu Val Leu Gly Gly Lys Arg Ala
145                 150                 155                 160

Val Lys Arg Gly Val Val Asn Leu Val Lys Tyr Gly Arg
                165                 170

(2) INFORMATION FOR SEQ ID NO: 7:

    (i) SEQUENCE CHARACTERISTICS:
        (A) LENGTH: 128 amino acids
        (B) TYPE: amino acid
        (C) STRANDEDNESS: single
        (D) TOPOLOGY: linear

    (ii) MOLECULE TYPE: protein

    (vi) ORIGINAL SOURCE:
        (A) ORGANISM: PORCINE REPRODUCTIVE AND RESPIRATORY SYNDROME
            VIRUS (PRRSV)
        (B) STRAIN: PRRS-OLOT

    (vii) IMMEDIATE SOURCE:
        (B) CLONE: PRRS-CY-JPD-P5-6-91

    (xi) SEQUENCE DESCRIPTION: SEQ ID NO: 7:

Met Ala Gly Lys Asn Gln Ser Gln Lys Lys Lys Lys Ser Ala Ala Pro
1               5                   10                  15

Met Gly Asn Gly Gln Pro Val Asn Gln Leu Cys Gln Leu Leu Gly Ala
            20                  25                  30

Met Ile Lys Ser Gln Arg Gln Gln Pro Arg Gly Gly Gln Ala Lys Lys
        35                  40                  45

Lys Lys Pro Glu Lys Pro His Phe Pro Leu Ala Ala Glu Asp Asp Ile
    50                  55                  60

Arg His His Leu Thr Gln Thr Glu Arg Ser Leu Cys Leu Gln Ser Ile
65                  70                  75                  80

Gln Thr Ala Phe Asn Gln Gly Ala Gly Thr Ala Ser Leu Ser Ser Ser
                85                  90                  95

Gly Lys Val Ser Phe Gln Val Glu Phe Met Leu Pro Val Ala His Thr
            100                 105                 110

Val Arg Leu Ile Arg Val Thr Ser Thr Ser Ala Ser Gln Gly Ala Ser
        115                 120                 125